# EUROPEAN PATENT APPLICATION

(11) **EP 2 460 879 A1**
(43) Date of publication of application: **06.06.2012**
(21) Application number: 12151812.0
(22) Date of filing: 16.07.2007
(51) Int. Cl.: C12N 9/14

(54) **Extended antegrade epicardial coronary infusion of adeno-associated viral vectors for gene therapy**

(30) Priority: 25.07.2006 US 83332406 P
(62) Divisional of application: 07810511.1
(71) Applicant: Celladon Corporation, La Jolla, California 92037 (US)
(72) Inventor: Zsebo, Krisztina Maria, Del Mar, CA 92014-3107 (US)
(74) Representative: Harrison, Susan Joan

(57) **Abstract**

The present invention relates to therapies for the treatment of cardiovascular diseases, particularly the delivery of therapeutic agents to heart tissue by direct infusion into the coronary circulation. A preferred embodiment of the invention is the use of a therapeutic polynucleotide composition adapted to transfect cardiac cells in the preparation of a medicament for treating or preventing cardiac disease by infusing the polynucleotide into a blood vessel of the coronary circulation over a period of at least three minutes, without isolating the coronary circulation from the systemic circulation. In a further preferred embodiment, the polynucleotide comprises a SERCA2a coding sequence packaged in a DNase resistant particle (DNP) of an AAV2/1 viral vector, the total number of DRP infused is less than or equal to 1 x 10¹³, and the blood vessel is the right or left coronary artery.

## Description

### CROSS-REFERENCE TO RELATED APPLICATIONS

This application claims the benefit of U.S. Provisional Application No. 60/833,324, filed July 25, 2006.

### BACKGROUND OF THE INVENTION

### Field of the Invention

The present invention relates to gene therapies for the treatment of cardiovascular diseases, particularly the delivery of polynucleotides to heart tissue.

### Description of the Related Art

Heart disease is a major public health issue of very high prevalence, especially in the Western world. Cardiac conditions include coronary artery disease, ischemic heart disease, heart failure, valvular heart disease, cardiac arrhythmias and cardiac inflammation (myocarditis) to name a few. Coronary artery disease and heart failure are possibly the most serious and prevalent, together being a leading cause of death in the Western world. The impact of acute myocardial infarction and congestive heart failure and their sequelae on the quality of life of patients and the cost of health care drives the search for new therapies.

Congestive heart failure (CHF) is a serious condition in which the heart loses its ability to pump blood efficiently. Data from the National Heart, Lung and Blood Institute, suggests about 5 million people in the United States alone have heart failure, and another 550,000 new cases are diagnosed each year. CHF contributes to or causes about 300,000 deaths annually. The disease is most common in people aged 65 or older, women and African Americans. The most common symptoms of heart failure are shortness of breath, feeling tired, and swelling in the ankles, feet, legs, and sometimes the abdomen. There is no cure for congestive heart failure, and a clear need exists in the art for effective therapies.

One method of treating heart disease, such as CHF, which has begun to receive more attention is gene therapy, wherein a polynucleotide is delivered to the cardiac tissue, typically in a viral vector. Numerous means of delivering viral vector to the heart have been attempted, including direct injection into the heart muscle (Liu et al., FASEB J. 2006; 20(2):207-16; Li et al. Toxicol. Appl. Pharmacol. 2006 Jan 25 (electronic publication); Zhu et al., Circulation. 2005; 112(17):2650-9), intracoronary delivery (Nykanen et al., Circ. Res. 2006; 98(11):1373-80; Kaspar et al., J. Gene Med. 2005; 7(3):316-24), catheter-based antegrade intracoronary delivery with coronary venous blockade (Hayase et al., Am. J. Physiol. Heart Circ. Physiol. 2005; 288(6):H2995-3000), aortic and pulmonary artery cross clamping followed by proximal aortic injection of adeno-associated viral vector (Kaspar et al., J. Gene Med. 2005; 7(3):316-24). Leiden and Svensson mention *in vivo* infusion of a rAAV vector into a coronary artery or sinus generally, but only describe in detail the perfusion of a mouse heart with a reporter gene *ex vivo* at 4°C where the heart has stopped beating (WO 00/38518) - a method that is impractical for the treatment of large animals, such as humans. Thus, these methods are all inadequate for use in a clinical setting, for example because these methods are too risky due to the need for surgical intervention or interruption of flow of oxygenated blood to the heart muscle, because of the amount of viral vector required to practice the method, because of the low percentage of tissue transfected, because the fact that transduction is limited to the site of the injection/administration only, or because the method is not practical or unproven for the treatment of disease in large animals or humans. There remains a need for a simple, minimally invasive, yet effective means of delivering transgenes in viral vectors to cardiac tissue to treat a disease, particularly in humans.

### SUMMARY OF THE INVENTION

The present invention relates to therapies for the treatment of cardiovascular diseases, particularly the delivery of therapeutic agents to heart tissue by direct epicardial infusion into the coronary circulation. A preferred embodiment of the invention is the use of a therapeutic polynucleotide composition adapted to transfect cardiac cells in the preparation of a medicament for treating or preventing cardiac disease by infusing the polynucleotide into a blood vessel of the coronary circulation over a period of at least three minutes, without isolating the coronary circulation from the systemic circulation.

Another embodiment is a method of treating or preventing a cardiovascular disease by transfecting cardiac cells of a large mammal, the method comprising: identifying an mammal in need of treatment or prevention of a cardiovascular disease; infusing a therapeutic polynucleotide into a blood vessel of the coronary circulation in vivo, where the therapeutic polynucleotide is infused into the blood vessel over a period of at least about three minutes, where the coronary circulation is not isolated or substantially isolated from the systemic circulation of the mammal; and where the therapeutic polynucleotide transfects cardiac cells of the mammal resulting in the treatment or prevention of the cardiovascular disease.

In some embodiments, the polynucleotide is infused into the blood vessel over a period of at least about five minutes, in some the polynucleotide is infused into the blood vessel over a period of at least about ten minutes, in some it is infused into the blood vessel over a period of at least about fifteen minutes.

In some embodiments the infusion into the blood vessel is at a rate of less than or equal to about 6.0 mL/min, in some it is at a rate of less than or equal to about 2.5 mL/min, in some it is at a rate of less than or equal to about 2.0 mL/min, in some it is at a rate of less than or equal to about 1.2 mL/min, in some it is at a rate of less than or equal to about 1.0 mL/min, in some is at a rate of less than or equal to about 0.6 mL/min.

In some embodiments, the blood vessel is the left coronary artery. In some embodiments the outflow of the coronary circulation is not nonnaturally restricted.

In some embodiments, the transfection of cardiac cells of the anterior lateral ventricle, inferior lateral ventricle, septum and right ventricle is detectable using PCR.

In some embodiments, the polynucleotide is capable of expressing a protein capable of modulating a cellular activity of the cardiac cells. In some embodiments, the cellular activity is a calcium cycling pathway of a cardiomyocyte. In some embodiments, the protein is a sarcoplasmic/endoplasmic reticulum ATPase (SERCA), and in some embodiments, the SERCA is SERCA2a.

In a preferred embodiment, the polynucleotide is present in a viral vector selected from the group consisting of an adeno-associated virus, an adenovirus, a retrovirus, a herpes simplex virus, a bovine papilloma virus, a lentiviral vector, a vaccinia virus, and a polyoma virus. In a more preferred embodiment, the viral vector is AAV virus, and in a more preferred embodiment the viral vector is an AAV2/1 vector. In some embodiments, the polynucleotide is operably linked to a CMV-based promoter and packaged in the viral vector. In a preferred embodiment, the polynucleotide comprises a SERCA2a coding sequence.

In some embodiments the transfection of the cardiac cells increases lateral ventricle fractional shortening. In some embodiments, the mammal is human and the disease is congestive heart failure.

In some embodiments, the polynucleotide is packaged in a DNase resistant particle (DRP) of a viral vector. In some embodiments, the total number of DRP infused is less than or equal to an amount selected from the group consisting of 1 x 10¹⁴ ,1 x 10¹³, 3 x 10¹², 1 x 10¹², 1 x 10¹¹, 1 x 10¹⁰, 1 x 10⁹, and 1 x 10⁸. In some embodiments, the total number of DRP infused is less than or equal to 1 x 10¹³.

In one embodiment, the total number of DRP infused is less than or equal to 1 x 10¹², the viral vector is AAV2/1, the polynucleotide comprises a SERCA2a coding sequence, the blood vessel is the left or right coronary artery, and the infusion of the polynucleotide lasts at least about 10 minutes at a flow rate of less than or equal to about 2 mL/min, and transfection of cardiac cells of the anterior lateral ventricle, inferior lateral ventricle, septum and right ventricle is detectable using PCR.

In some embodiments, the disease is selected from the group consisting of heart failure, ischemia, arrhythmia, myocardial infarction, congestive heart failure, transplant rejection, abnormal heart contractility, non-ischemic cardiomyopathy, mitral valve regurgitation, aortic stenosis or regurgitation, abnormal Ca²⁺ metabolism and congenital heart disease.

In one embodiment the viral vector is AAV2/1, the polynucleotide comprises a SERCA2a coding sequence, the blood vessel is the left or right coronary artery, and the infusion of the polynucleotide lasts at least about 8 minutes at a flow rate of less than or equal to about 2.5 mL/min, where transfection of cardiac cells of the anterior lateral ventricle, inferior lateral ventricle, septum and right ventricle is detectable using PCR, and the transfection of the cardiac cells increases lateral ventricle fractional shortening when measured about 4 months after the infusion by at least 25% as compared to lateral ventricle fractional shortening before infusion of the polynucleotide.

In one embodiment, the mammal is a human, the viral vector is AAV2/1, the polynucleotide comprises a SERCA2a coding sequence, the blood vessel is the left or right coronary artery, and the infusion of the polynucleotide lasts at least about 10 minutes. In some embodiments, the disease of the cardiovascular system is congestive heart failure. In some embodiments, the infusion of the polynucleotide is at a flow rate of about 6 mL/min.

In some embodiments, the transfection of the cardiac cells results in an improvement in a measure of cardiac function selected from the group consisting of expression of SERCA2a protein, fractional shortening, ejection fraction, cardiac output, time constant of ventricular relaxation, and regurgitant volume.

In some embodiments, the invention further comprises infusing a polynucleotide into an additional blood vessel of the coronary circulation where the polynucleotide is infused into the additional blood vessel over a period of at least about three minutes. In some embodiments, the mammal is a human, the viral vector is AAV2/1, the polynucleotide comprises a SERCA2a coding sequence, the blood vessel is the left coronary artery, and the infusion of the polynucleotide into the left coronary artery lasts at least about 6 minutes, and the additional blood vessel is the right coronary artery, and the infusion of the polynucleotide into the right coronary artery lasts at least about 3 minutes. In some embodiments, the disease of the cardiovascular system is congestive heart failure. In some embodiments, the infusion of the polynucleotide into the left coronary artery and the right coronary artery is at a flow rate of about 6 mL/min.

In some embodiments, the transfection of the cardiac cells results in an improvement in a measure of cardiac function selected from the group consisting of expression of SERCA2a protein, fractional shortening, ejection fraction, cardiac output, time constant of ventricular relaxation, and regurgitant volume.

In some embodiments, the invention is kit comprising: a pharmaceutical composition comprising at least 1 x 10¹¹ DRP of AAV2/1 vector containing a polynucleotide encoding for SERCA2a; and instructions instructing that a solution comprising at least 0.5 x 1011 DRP of AAV2/1 vector containing a polynucleotide encoding for SERCA2a should be administered to a patient in need of prevention or treatment of a cardiovascular disease by in vivo infusion into a blood vessel of the coronary circulation where the coronary circulation is not isolated or substantially isolated from the systemic circulation of the patient, and where the polynucleotide is infused into the blood vessel over a period of at least about three minutes. In some embodiments, the blood vessel is the left or right coronary artery, and the infusion of the polynucleotide is for a period of at least about eight minutes.

### BRIEF DESCRIPTION OF THE DRAWINGS

Figure 1 is a schematic showing the features of the AAV2/1/SERCA2a (tgABG12) DNA.

Figure 2 is a schematic comparison of wild-type and recombinant AAV DNA.

Figure 3 is an illustration of the heart showing the location of samples collected in the experiment described in Example 1.

Figure 4 shows the location of the PCR primers used in the detection of the AAV2/1/SERCA2a DNA in tissue samples.

Figure 5 is a chart showing the results of the experiment described in Example 1, indicating that increased infusion time of the same amount of AAV2/1/SERCA2a resulted in increased copies of AAV2/1/SERCA2a in the samples of heart tissue.

Figure 6 is a chart showing the results of the experiment described in Example 2, indicating that infusion of AAV2/1/SERCA2a resulted in greater copies of AAV2/1/SERCA2a than i.v. injection in a dose dependent manner.

Figure 7A is polyacrylamide gels showing the expression of SERCA2a protein (top) and mRNA (bottom) in non-experimental control animals, formulation infused animals (saline) and AAV2/1/SERCA2a treated animals from Example 3; Figure 7B is a graph comparing the normalized protein expression of SERCA2a between the three treatment groups of Example 3.

Figure 8 is a graph showing the results of the experiment described in Example 3, indicating that antegrade epicardial coronary infusion of AAV2/1/SERCA2a results in increased fractional shortening of the left ventricle, a measure of improved heart function in a model of heart failure.

Figure 9 is a plot of the absolute change in fractional shortening on day 112 compared to day 56.

Figure 10 is a plot of the absolute change in ejection fraction on day 112 compared to day 56.

Figure 11 is a plot of the absolute change in cardiac output (mL/min.) on day 112 compared to day 56.

Figure 12 is a plot of the absolute change in tau (time constant of LV relaxation in msec.) on day 112 compared to day 56.

Figure 13 is a plot showing the absolute change in regurgitant volume (mL) on day 112 compared to day 56.

### DETAILED DESCRIPTION OF THE PREFERRED EMBODIMENT

The present invention relates to therapies for the treatment of cardiovascular diseases. One embodiment of the invention is the novel use of a therapeutic polynucleotide composition adapted to transfect cardiac cells in the preparation of a medicament for treating heart disease by transfecting heart tissue to improve cardiac contractility of patients with congestive heart failure (CHF) by normalizing cardiomyocyte calcium levels. The preferred embodiment uses a gene transfer agent, AAV2/1/SERCA2a, infused *in* vivo directly into the coronary circulation for a period of time not less than about three minutes, where the coronary circulation is not isolated from the systemic circulation of the animal or otherwise nonnaturally restricted.

As used herein, "polynucleotide " has its ordinary and customary meaning in the art and includes any polymeric nucleic acid such as DNA or RNA molecules, as well as chemical derivatives known to those skilled in the art. Polynucleotides include not only those encoding a therapeutic protein, but also include sequences that can be used to decrease the expression of a targeted nucleic acid sequence using techniques known in the art (e.g., antisense, interfering, or small interfering nucleic acids). One example is a sequence which reduces or eliminates the expression of phospholamban. Polynucleotides can also be used to initiate or increase the expression of a targeted nucleic acid sequence or the production of a targeted protein within cells of the cardiovascular system. Targeted nucleic acids and proteins include, but are not limited to, nucleic acids and proteins normally found in the targeted tissue, derivatives of such naturally occurring nucleic acids or proteins, naturally occurring nucleic acids or proteins not normally found in the targeted tissue, or synthetic nucleic acids or proteins. One or more polynucleotides can be used in combination, administered simultaneously and/or sequentially, to increase and/or decrease one or more targeted nucleic acid sequences or proteins.

As used herein the terms "infusion," "infused," and "infusing" have their ordinary and customary meaning in the art and refer to administration for a time period (typically a minute or more) that is substantially longer than the art recognized term of "injection" or "bolus injection," (typically less than a minute). The flow rate of the infusion will depend at least in part on the volume administered, however the flow rate of an "infusion" is slower than that of an "injection" for the same volume.

An "effective amount" has its ordinary and customary meaning in the art and includes an amount sufficient to effect or achieve a beneficial or desired therapeutic effect. For example, an "effective amount" is an amount that achieves any of the following: an increase in lateral ventricle fractional shortening; and/or palliation, amelioration, stabilization, reversal, slowing or delay in the progression or a sign or symptom of the disease state. An effective amount can be administered in one or more administrations.

As used herein "in conjunction with," "in combination with," "concurrent," or "concurrently," have their ordinary and customary meaning in the art and include administration of one treatment modality in addition to another treatment modality. For example, infusion of a polynucleotide of the present invention to a subject in addition to administration of art recognized pharmaceutical composition to the same individual. As used herein, these terms include simultaneous administration, as well as administration of the treatment modalities sequentially.

As used herein, "treat" or "treatment" of disease has its ordinary and customary meaning in the art and includes the stabilization, cure, or less than complete cure of a disease, including the halting or slowing of the progression of a disease or a sign or symptom of the disease. The term "prevention" has its ordinary and customary meaning in the art and includes complete or incomplete prevention, or a delay of the onset of, a disease or a sign or symptom of a disease. The terms "therapeutic," "therapeutic effect" or "clinical effect" include both treatment and prevention. Examples of diseases intended to be treated using the present invention that are associated with the cardiovascular system include, but are not limited to, heart failure, ischemia, arrhythmia, myocardial infarction, congestive heart failure, transplant rejection, abnormal heart contractility, non-ischemic cardiomyopathy, mitral valve regurgitation, aortic stenosis or regurgitation, abnormal Ca²⁺ metabolism and congenital heart disease. For example, beneficial or desired clinical results or therapeutic effects include, but are not limited to, a greater alleviation of signs or symptoms of cardiovascular disease, increased diminishment of extent of disease, stabilization (i.e., not worsening) of disease state, delay or slowing of disease progression, amelioration or palliation of the disease state, and remission (whether partial or total), whether detectable or undetectable. Other examples of therapeutic effect include, but are not limited to, increased lateral ventricle fractional shortening; augmented cardiac contractility at the cellular and intact animal levels, reversal of cardiac remodeling, and normalization of the abnormally high diastolic levels of cytosolic calcium. Other clinical features which can be improved in a subject treated with an embodiment of the present invention include without limitation survival, cardiac metabolism, heart contractility, heart rate, ventricular function (e.g., left ventricular end-diastolic pressure (LVEDP), left ventricular systolic pressure (LVSP)), Ca²⁺ metabolism (e.g., intracellular Ca²⁺ concentration, peak or resting [Ca²⁺], SR Ca²⁺ ATPase activity, phosphorylation state of phospholamban), force generation, relaxation and pressure of the heart, a force frequency relationship, cardiocyte survival or apoptosis or ion channel activity (e.g., sodium calcium exchange, sodium channel activity, calcium channel activity, sodium potassium ATPase pump activity), activity of myosin heavy chain, troponin I, troponin C, troponin T, tropomyosin, actin, myosin light chain kinase, myosin light chain 1, myosin light chain 2 or myosin light chain 3, IGF-1 receptor, PI3 kinase, AKT kinase, sodium-calcium exchanger, calcium channel (L and T), calsequestrin or calreticulin. Other measures of cardiac disease which can be improved include fractional shortening, cardiac output, ejection fraction, Tau, regurgitant volume, reduced hospital stays, improved quality of life, and increased treadmill time.

As used herein, "exogenous" nucleic acids or genes are those that do not occur in nature in the vector utilized for nucleic acid transfer; e.g., not naturally found in the viral vector, but the term is not intended to exclude nucleic acids encoding a protein or polypeptide that occurs naturally in the patient or host, e.g., SERCA.

As used herein, "cardiac cell" includes any cell of the heart that is involved in maintaining a structure or providing a function of the heart such as a cardiac muscle cell, a cell of the cardiac vasculature, or a cell present in a cardiac valve. Cardiac cells include cardiomyocytes (having both normal and abnormal electrical properties), epithelial cells, endothelial cells, fibroblasts, cells of the conducting tissue, cardiac pacemaking cells, and neurons.

As used herein, "isolated," "substantially isolated" or "largely isolated" and their variants are terms that do not require complete or absolute isolation of the coronary venous, cardiac, systemic venous, or systemic circulation; rather, they are intended to mean that a majority, preferably the major part or even substantially all of the specified circulation is isolated. As used herein, "partially isolated" refers to any nontrivial portion of the specified circulation being isolated.

As used herein, "nonnaturally restricted" includes any method of restricting the flow of fluid through a blood vessel, e.g., balloon catheter, sutures, etc., but does not include naturally occurring restriction, e.g. plaque build-up (stenosis). Nonnatural restriction includes substantial or total isolation of, for example, the coronary circulation.

As used herein, "modulating" has its ordinary meaning, and encompasses both increasing and decreasing the expression or activity of the target.

As used herein, the term "minimally invasive" is intended to include any procedure that does not require open surgical access to the heart or vessels closely associated with the heart. Such procedures include the use of endoscopic means to access the heart, and also catheter-based means relying on access via large arteries and veins, such as the femoral artery.

As used herein, the term "adeno-associated virus" or "AAV" encompasses all subtypes, serotypes and pseudotypes, as well as naturally occurring and recombinant forms. A variety of AAV serotypes and strains are known in the art and are publicly available from sources, such as the ATCC, and academic or commercial sources. Alternatively, sequences from AAV serotypes and strains which are published and/or available from a variety of databases may be synthesized using known techniques.

As used herein, the term "serotype" refers to an AAV which is identified by and distinguished from other AAVs based on capsid protein reactivity with defined antisera. There are at least twelve known serotypes of human AAV, including AAV1 through AAV12, however additional serotypes continue to be discovered, and use of newly discovered serotypes are contemplated. For example, AAV2 serotype is used to refer to an AAV which contains capsid proteins encoded from the cap gene of AAV2 and a genome containing 5' and 3' inverted terminal repeat (ITR) sequences from the same AAV2 serotype.

A "pseudotyped" AAV refers to an AAV that contains capsid proteins from one serotype and a viral genome including 5' and 3' inverted terminal repeats (ITRs) of a different or heterologous serotype. A pseudotyped rAAV would be expected to have cell surface binding properties of the capsid serotype and genetic properties consistent with the ITR serotype. A pseudotype rAAV may comprise AAV capsid proteins, including VP1, VP2, and VP3 capsid proteins, and ITRs from any serotype AAV, including any primate AAV serotype from AAV1 through AAV12, as long as the capsid protein is of a serotype heterologous to the serotype(s) of the ITRs. In a pseudotype rAAV, the 5' and 3' ITRs may be identical or heterologous. Pseudotyped rAAV are produced using standard techniques described in the art.

A "chimeric" rAAV vector encompasses an AAV vector comprising heterologous capsid proteins; that is, a rAAV vector may be chimeric with respect to its capsid proteins VP1, VP2 and VP3, such that VP1, VP2 and VP3 are not all of the same serotype AAV. A chimeric AAV as used herein encompasses AAV wherein the capsid proteins VP1, VP2 and VP3 differ in serotypes, including for example but not limited to capsid proteins from AAV1 and AAV2; are mixtures of other parvo virus capsid proteins or comprise other virus proteins or other proteins, such as for example, proteins that target delivery of the AAV to desired cells or tissues. A chimeric rAAV as used herein also encompasses a rAAV comprising chimeric 5' and 3' ITRs. The present invention encompasses chimeric rAAV vectors that comprise ITRs from different AAV serotypes, for example AAV1 and AAV2, or a chimeric rAAV may comprise synthetic sequences.

rAAV viral vectors may be produced by any of a number of methods known in the art including transient transfection strategies as described in U.S. Pat. Nos. 6,001,650 and 6,258,595. Typically, rAAV vector production requires four common elements: 1) a permissive host cell for replication which includes standard host cells known in the art including 293-A, 293-S (obtained from BioReliance), VERO, and HeLa cell lines which are applicable for the vector production systems described herein; 2) helper virus function which is supplied as a plasmid, pAd Helper 4.1 expressing the E2a, E4-orf6 and VA genes of adenovirus type 5 (Ad5) when utilized in transduction production systems; 3) a transpackaging rep-cap construct; and 4) a gene of interest flanked by AAV ITR sequences. Transfection production may be performed as described in the article by Sandalon et al., J. Virology, 2004; 78(22):12355-12365.

### Treatment Method

In a preferred embodiment of the invention, the therapeutic agent, e.g. polynucleotide/viral vector described in more detail below, is administered to the subject by infusion into a blood vessel of the coronary circulation of the beating heart *in vivo* for a period of at least about three minutes in a particular blood vessel. In large animal models of the human heart and cardiovascular disease, Applicants have found that, unexpectedly, for administration of viral vectors a relatively long infusion time is more effective and results in superior gene transfer efficiency into heart tissue than a bolus injection or short (e.g., ≤1 minute) infusion time of the same amount of viral vector. The improved effectiveness of infusion can be measured as a greater copy number of the transgene per cell, increased expression of the transgene at the mRNA and/or protein level per cell or in the tissue, and/or a greater percentage of cells of a particular tissue, e.g. cardiomyocytes, being transfected, as compared to injection. Applicants have shown that in large animal models, this results in successful treatment of models of human cardiovascular disease. In addition, Applicants have discovered that by using relatively long infusion times, there is no need to isolate the coronary circulation from the systemic circulation or otherwise re-circulate the therapeutic agent, or to artificially restrict the coronary venous circulation as a means to increase pressure within the coronary circulation or to increase dwell time of the therapeutic agent. Nor is there any need to pretreat the heart tissue with other agents such as vasoactive agents (e.g., histamine, histamine agonists, or a vascular endothelial growth factor), to cool the heart, stop the heart, or remove the heart from the animal for perfusion. Instead, Applicants' procedure can be practiced in a standard catheterization lab setting using existing catheters for administration. Thus, Applicants have discovered a simple, practical, and efficacious means of using gene therapy to treat cardiovascular disease in large animals, such as humans.

In a preferred embodiment of the invention, the therapeutic agent is administered to the subject by infusion into a blood vessel of the coronary circulation. The coronary circulation provides blood supply to the tissue of the heart. There are a number of coronary arteries. Normally, four main coronary arteries provide oxygenated blood to the heart for distribution throughout the heart tissue; the left main and right coronary arteries, the left anterior descending artery, and the left circumflex artery. Infusion of one or a combination of these arteries is contemplated, for example infusion of the left and right coronary arteries. The preferred embodiment utilizes antegrade, epicardial infusion of the left and right main coronary arteries. Also contemplated is retrograde infusion of a coronary artery, or a combination of one or more antegrade and retrograde coronary arteries or veins. Infusion of the coronary blood vessel(s) is performed using standard guidewires, catheters and infusion pumps. In a preferred embodiment, the infusion catheter is directed to the coronary artery under fluoroscopic guidance via the femoral artery. As used herein, "blood vessel of the coronary circulation," "coronary blood vessel" or "blood vessel of the heart" includes grafts onto coronary blood vessels, for example those resulting from bypass surgery. As used herein, "epicardial" refers to blood vessels located on the outer portion of the heart, e.g. the left or right coronary arteries.

Once the infusion catheter is in place in the target coronary blood vessel, the therapeutic agent is infused into the blood vessel, preferably by means of a programmable infusion pump. The amount of time taken to infuse the therapeutic agent is an important factor in obtaining effective and superior gene transfer efficiency. Applicants have determined that an infusion time of at least about 3 minutes into a particular blood vessel is more effective than a bolus injection or shorter infusion time. Preferably, the infusion time is at least about 8 minutes, more preferably at least about 10 minutes, although infusion times of at least about 15 minutes are contemplated. Applicants also contemplate that the infusion time is, is about, is at least, is at least about, is not more than, or is not more than about, 1, 2, 3, 4, 5, 6, 7, 8, 9, 10, 11, 12, 13, 14, 15, 16, 17, 18, 19, or 20 minutes, or falls within a range defined by any two of these values.

Because the infusion typically involves the use of a catheter and connecting tubing which has a certain dead volume, the infusion device is often primed with a carrier solution, e.g. blood from the subject, which does not contain any therapeutic agent. Thus, the therapeutic agent is not immediately administered into the coronary circulation when the infusion pump is turned on. Likewise, when the syringe containing the therapeutic agent is emptied, an amount of therapeutic agent typically remains in the dead volume of the connecting tubing and catheter. Immediately following the infusion of the therapeutic agent, the dead volume is flushed with an appropriate solution, preferably at the same flow rate used to administer the therapeutic agent. The period of time over which the therapeutic agent is actually being delivered into the coronary circulation, as opposed to displacing dead volume in the infusion apparatus, is the "infusion time" referred to above. For example, if 3 mL of therapeutic agent is loaded into an infusion apparatus with 3 mL of dead volume, and the infusion rate is 1 mL/min., the time required to infuse the therapeutic agent into the coronary circulation is only 3 minutes, while the total time required to administer the 3 mL of therapeutic agent and 3 mL of dead volume is 6 minutes. In some embodiments, the catheter and any connecting tubing is primed with the therapeutic agent such that the dead volume is not an issue. Similarly, the effective amount of therapeutic agent could be delivered without the need to flush the tubing. However, this results in therapeutic agent being left in the tubing, wasting the therapeutic agent.

Applicants contemplate that the therapeutic agent will be infused at a flow rate that is, is about, is at least, is at least about, is not more than, or is not more than about, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0, 5.5, 6.0, 6.5, 7.0, 7.5, 8.0, 8.5, 9.0, 9.5, or 10.0 mL/min., or falls within a range defined by any two of these values. Preferably, the flow rate is between about 0.2 mL/min and about 6.0 mL/min., more preferably between about 0.2 mL/min and about 2.5 mL/min., more preferably between about 0.2 mL/min. and about 2.0 mL/min. Those of skill in the art will recognize that delivery of the therapeutic agent is possible without an infusion pump, however more accurate flow rates and uniform delivery are possible with the use of an infusion pump.

The total amount of viral particles or DNase resistant particles (DRP) delivered by infusion to provide an effective amount is preferably between 1 x 10¹⁴ and about 1 x 10¹¹, more preferably between about 3 x 10¹² and 1 x 10¹², and more preferably about 1 x 10¹². However, applicants also contemplate that the total amount of viral particles or DRP is, is about, is at least, is at least about, is not more than, or is not more than about, 1 x 10¹⁴, 9 x 10¹³, 8 x 10¹³, 7 x 10¹³, 6 x 10¹³, 5 x 10¹³, 4 x 10¹³, 3 x 10¹³, 2 x 10¹³, 1 x 10¹³, 9 x 10¹², 8 x 10¹², 7 x 10¹², 6 x 10¹², 5 x 10¹², 4 x 10¹², 3 x 10¹², 2 x 10¹², 1 x 10¹², 9 x 10¹¹, 8 x 10¹¹, 7 x 10¹¹, 6 x 10¹¹, 5 x 10¹¹, 4 x 10¹¹, 3 x 10¹¹, 2 x 10¹¹, 1 x 10¹¹, 9 x 10¹⁰, 8 x 10¹⁰, 7 x 10¹⁰, 6 x 10¹⁰, 5 x 10¹⁰, 4 x 10¹⁰, 3 x 10¹⁰, 2 x 10¹⁰, 1 x 10¹⁰, 9 x 10⁹, 8 x 10⁹, 7 x 10⁹, 6 x 10⁹, 5 x 10⁹, 4 x 10⁹, 3 x 10⁹, 2 x 10⁹, 1 x 10⁹, 9 x 10⁸, 8 x 10⁸, 7 x 10⁸, 6 x 10⁸, 5 x 10⁸, 4 x 10⁸, 3 x 10⁸, 2 x 10⁸, or 1 x 10⁸, or falls within a range defined by any two of these values.

The number of DRP infused over a given time is a function of the concentration of the solution being infused and the flow rate. The rate of DRP or viral particle infusion is preferably between about 1 x 10⁸/min. and about 1 x 10¹⁴/min., more preferably between about 5 x 10¹⁰/min. and about 5 x 10¹²/min., more preferably between about 3 x 10¹⁰/min. and about 1 x 10¹²/min., more preferably between about 6 x 10¹⁰/min. and about 4 x 10¹¹/min. In a preferred embodiment, the rate of DRP or viral particle infusion is 1 x 10¹¹/min., and in another preferred embodiment, it is 1.25 x 10¹¹/min.

In one embodiment, the therapeutic agent is administered into a single blood vessel of the heart. In another embodiment 2/3 of the total volume of therapeutic agent is delivered to one blood vessel of the heart, and 1/3 is administered to another blood vessel of the heart. In another embodiment, more than 2 coronary blood vessels are infused, (e.g. 3, 4, 5 or more), and the portion of total infusion volume containing the therapeutic agent administered per blood vessel can be adjusted as appropriate. The goal of the infusion is to provide diffuse, homogenous myocardial exposure to AAV2/1/SERCA2a via anterograde, epicardial coronary infusion. Multiple infusion scenarios exist based on collateralization patterns, occlusive disease, and anatomic variation (e.g. post surgical bypass anatomy), but the clinician's goal is 1/3 of AAV2/1/SERCA2a delivered to the anterolateral, 1/3 delivered to the posterolateral, and 1/3 delivered to the inferior/inferolateral myocardium. Anatomy is defined by coronary and bypass graft angiography to accomplish homogenous delivery to the perfused myocardium. In addition, one of skill in the art will recognize that while sheep and pigs are accepted animal models for human cardiovascular studies, sheep and pigs are 90% left dominant. In comparison, approximately ~10% of the human population is left dominant, with the remaining 90% being right or co-dominant (Vlodaver Z. et al. Coronary Heart Disease: Clinical, Angiographic, and Pathologic Profiles. Spinger-Verlag, New York. 1976). One pathologic series suggests that 71% of patients are right dominant, 17% co-dominant, 12% are left dominant (McAlpine W. Heart and Coronary Arteries. Spinger Verlag, 1975). Therefore, to achieve a similar perfusion of the left ventricle in humans vs. pigs/sheep, the optimum infusion scenario can differ.

A 1/3 and 2/3 split of the solution volume is preferred for two blood vessels, however the portion of the injection volume infused into a particular blood vessel can be a volume that is, is about, is at least, is at least about, is not more than, or is not more than about, 20, 25, 30, 35, 40, 45, 50, 55, 60, 65, 70, 75, or 80% of the total volume, or falls within a range defined by any two of these values. The total volume of solution containing the therapeutic agent will vary according to the size of the animal being treated. For a human subject, a total therapeutic agent volume of 60 mL is preferred. However the total volume of therapeutic agent can be a volume that is, is about, is at least, is at least about, is not more than, or is not more than about, 1, 2, 3, 4, 5, 10, 15, 20, 25, 30, 35, 40, 45, 50, 55, 60, 65, 70, 75, 80, 85, 90, 95, 100, 105, 110, 115, 120, 125, 130, 135, 140, 145, or 150 mL, or falls within a range defined by any two of these values.

The therapeutic agents described herein can be in solution, preferably a pharmaceutical composition suitable for administration directly into the coronary circulation. The ingredients of an acceptable pharmaceutical composition are known to those of skill in the art, and can include such elements as a buffer and suitable carrier. In another embodiment, the pharmaceutical composition containing a therapeutic agent, for example a viral vector, and more preferably a AAV2/1/SERCA2a vector, is part of a kit. In some embodiments, the kit contains a stock solution of therapeutic agent and a solution for diluting the stock solution. Also included in the kit are instructions for administration of the viral vector, preferably by infusion directly into the coronary circulation as described in any of the embodiments disclosed herein. Similarly, the therapeutic agents described herein can be used in the manufacture of a medicament for the treatment of the disease disclosed herein, where the medicament is infused directly into the cardiac circulation.

### Methods of Polynucleotide Delivery

One aspect of the present invention contemplates transfer of therapeutic polynucleotide into a cell. Such transfer may employ viral or non-viral methods of gene transfer. This section provides a discussion of methods and compositions of gene or nucleic acid transfer, including transfer of antisense, interfering, and small interfering sequences.

In one embodiment, the therapeutically significant polynucleotides are incorporated into a viral vector to mediate transfer to a cell. Additional expression constructs encoding other therapeutic agents as described herein may also be transferred via viral transduction using infectious viral particles, for example, by transformation with an adeno-associated virus (AAV) of the present invention. Alternatively, a retrovirus, bovine papilloma virus, an adenovirus vector, a lentiviral vector, a vaccinia virus, a polyoma virus, or an infective virus that has been engineered to express may be used. Similarly, nonviral methods which include, but are not limited to, direct delivery of DNA such as by perfusion, naked DNA transfection, liposome mediated transfection, encapsulation, and receptor-mediated endocytosis may be employed. These techniques are well know to those of skill in the art, and the particulars thereof do not lie at the crux of the present invention and are thus need not be exhaustively detailed herein. However, in one preferred example, a viral vector is used for the transduction of cardiac cells to deliver a therapeutically significant polynucleotide to a cell. The virus may gain access to the interior of the cell by a specific means such receptor-mediated endocytosis, or by non-specific means such as pinocytosis.

A number of exemplary vectors will now be described. It will be appreciated that the following discussion is non-exhaustive.

### Adeno-associated Virus Vectors

A preferred embodiment of the invention utilizes purified, replication incompetent, pseudotyped recombinant adeno-associated viral (rAAV) particles. Adeno-associated viruses (AAV) are parvoviruses belonging to the genus *Dependovirus.* They are small, nonenveloped, single-stranded DNA viruses which require a helper virus in order to replicate. Co-infection with a helper virus (e.g., adenovirus, herpes virus, or vaccinia virus) is necessary in order to form functionally complete AAV virions. *In vitro,* in the absence of co-infection with a helper virus, AAV establishes a latent state in which the viral genome exists in an episomal form, but infectious virions are not produced. Subsequent infection by a helper virus "rescues" the genome, allowing it to be replicated and packaged into viral capsids, thereby reconstituting the infectious virion. Recent data indicate that *in vivo* both wild type AAV and recombinant AAV predominantly exist as large episomal concatemers.

AAV are not associated with any known human diseases, are generally not considered pathogenic, and do not appear to alter the physiological properties of the host cell upon integration. AAV can infect a wide range of host cells, including non-dividing cells, and can infect cells from different species. In contrast to some vectors, which are quickly cleared or inactivated by both cellular and humoral responses, AAV vectors have shown persistent expression in various tissues *in vivo.* The persistence of recombinant AAV vectors in non-diving cells *in vivo* may be attributed to the lack of native AAV viral genes and the vector's ability to form episomal concatemers.

Adeno-associated virus (AAV) is an attractive vector system for use in the cell transduction of the present invention as it has a high frequency of persistence as an episomal concatemer and it can infect non-dividing cells, thus making it useful for delivery of genes into mammalian cells, for example, in tissue culture and *in vivo.* Studies demonstrating the use of AAV in gene delivery include Flotte et al., Proc. Natl. Acad. Sci. USA, 1993; 90:10613-17 and Walsh et al., J. Clin. Invest., 1994; 94:1440-48. Recombinant AAV vectors have been used successfully for *in vitro* and *in vivo* transduction of marker genes and genes involved in human diseases (see for example, Walsh et al., J. Clin. Invest. 1994; 94:1440-48). AAV has a broad host range for infectivity. Details concerning the generation and use of rAAV vectors are described in U.S. Pat. No. 5,139,941 and/or U.S. Pat. No. 4,797,368.

Typically, recombinant AAV (rAAV) virus is made by cotransfecting a plasmid containing the gene of interest flanked by the two AAV terminal repeats and/or an expression plasmid containing the wild-type AAV coding sequences without the terminal repeats, for example pIM45. The cells are also infected and/or transfected with adenovirus and/or plasmids carrying the adenovirus genes required for AAV helper function. rAAV virus stocks made in such fashion are contaminated with adenovirus which must be physically separated from the rAAV particles (for example, by cesium chloride density centrifugation or column chromatography). Alternatively, adenovirus vectors containing the AAV coding regions and/or cell lines containing the AAV coding regions and/or some or all of the adenovirus helper genes could be used. Cell lines carrying the rAAV DNA as an integrated provirus can also be used.

Multiple serotypes of AAV exist in nature, with at least twelve serotypes (AAV1-AAV12) currently known. Despite the high degree of homology, the different serotypes have tropisms for different tissues. The receptor for AAV1 is unknown; however, AAV1 is known to transduce skeletal and cardiac muscle more efficiently than AAV2. Since in most of the studies have been done with pseudotyped vectors in which the vector DNA flanked with AAV2 ITR is packaged into capsids of alternate serotypes, it is clear that the biological differences are related to the capsid rather than to the genomes. Recent evidence indicates that DNA expression cassettes packaged in AAV1 capsids are at least 1 log₁₀ more efficient at transducing cardiomyocytes than those packaged in AAV2 capsids.

### Adenoviral Vectors

Another method for delivery of the polynucleotide for gene therapy involves the use of an adenovirus expression vector. "Adenovirus expression vector" is meant to include those constructs containing adenovirus sequences sufficient (a) to support packaging of the construct and/or (b) to ultimately express a tissue and/or cell-specific construct that has been cloned therein.

In one form of the invention, the expression vector comprises a genetically engineered form of adenovirus. Knowledge of the genetic organization of adenovirus, a 36 kb, linear, double-stranded DNA virus, allows substitution of large pieces of adenoviral DNA with foreign sequences up to 7 kb (Grunhaus and Horwitz, Seminar in Virology, 1992; 3:237-252). In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification.

Adenovirus growth and manipulation is known to those of skill in the art, and exhibits broad host range *in vitro* and *in vivo.* This group of viruses can be obtained in high titers, e.g., 10⁹ to 10¹¹ plaque-forming units per mL, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus, demonstrating their safety and/or therapeutic potential as *in vivo* gene transfer vectors.

Adenovirus vectors have been used in eukaryotic gene expression and vaccine development. Recently, animal studies suggested that recombinant adenovirus could be used for gene therapy (see, e.g., Stratford-Perricaudet et al., Hum. Gene. Ther., 1991;1:242-256; Rich *et al.,* 1993). Studies in administering recombinant adenovirus to different tissues include muscle injection, peripheral intravenous injections and stereotactic inoculation into the brain. Recombinant adenovirus and adeno-associated virus can both infect and transduce non-dividing human primary cells.

While the use of adenovirus vectors is contemplated, such use in cardiovascular gene therapy trials is currently limited by short-lived transgene expression. (Vassalli G, et al., Int. J. Cardiol., 2003; 90(2-3):229-38). This is due to cellular immunity against adenoviral antigens. Improved "gutless" adenoviral vectors have reduced immunogenicity, yet still are ineffective if maximal expression of the transgene for more than six months is needed or desired for therapeutic effect (Gilbert R, et al., Hum. Mol. Genet., 2003; 12(11):1287-99). AAV vectors have demonstrated long term expression (> 1 year) and are the preferred vector for therapeutic effects where expression is needed long-term (Daly TM, et al., Gene Ther., 2001; 8(17):1291-8).

### Retroviral Vectors

Retroviruses may be chosen as gene delivery vectors due to their ability to integrate their genes into the host genome, transferring a large amount of foreign genetic material, infecting a broad spectrum of species and cell types and for being packaged in special cell-lines.

The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome.

In order to construct a retroviral vector, a nucleic acid encoding a gene of interest is inserted into the viral genome in the place of certain viral sequences to produce a virus that is replication-defective. In order to produce virions, a packaging cell line is constructed containing the gag, pol, and/or env genes but without the LTR and/or packaging components. When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media. The media containing the recombinant retroviruses is then collected, optionally concentrated, and used for gene transfer. Retroviral vectors are able to infect a broad variety of cell types. However, integration and stable expression require the division of host cells.

### Herpes virus

Because herpes simplex virus (HSV) is neurotropic, it has generated considerable interest in treating nervous system disorders. Moreover, the ability of HSV to establish latent infections in non-dividing neuronal cells without integrating into the host cell chromosome or otherwise altering the host cell's metabolism, along with the existence of a promoter that is active during latency makes HSV an attractive vector. And though much attention has focused on the neurotropic applications of HSV, this vector also can be exploited for other tissues given its wide host range.

Another factor that makes HSV an attractive vector is the size and organization of the genome. Because HSV is large, incorporation of multiple genes or expression cassettes is less problematic than in other smaller viral systems. In addition, the availability of different viral control sequences with varying performance (temporal, strength, etc.) makes it possible to control expression to a greater extent than in other systems. It also is an advantage that the virus has relatively few spliced messages, further easing genetic manipulations.

HSV also is relatively easy to manipulate and can be grown to high titers. Thus, delivery is less of a problem, both in terms of volumes needed to attain sufficient multiplicity of infection (MOI) and in a lessened need for repeat dosing. For a review of HSV as a gene therapy vector, see Glorioso et al., Annu. Rev. Microbiol., 1995; 49:675-710. Avirulent variants of HSV have been developed and are readily available for use in gene therapy contexts (U.S. Pat. No. 5,672,344).

### Lentiviral Vectors

Lentiviruses are complex retroviruses, which, in addition to the common retroviral genes gag, pol, and env, contain other genes with regulatory or structural function. The higher complexity enables the virus to modulate its life cycle, as in the course of latent infection. Some examples of lentivirus include the Human Immunodeficiency Viruses (HIV-1, HIV-2) and the Simian Immunodeficiency Virus (SIV). Lentiviral vectors have been generated by multiply attenuating the HIV virulence genes, for example, the genes env, vif, vpr, vpu and nef are deleted making the vector biologically safe.

Lentiviral vectors are known in the art, see U.S. Pat. Nos. 6,013,516 and 5,994,136. In general, the vectors are plasmid-based or virus-based, and are configured to carry the essential sequences for incorporating foreign nucleic acid, for selection and for transfer of the nucleic acid into a host cell. The gag, pol and env genes of the vectors of interest also are known in the art. Thus, the relevant genes are cloned into the selected vector and then used to transform the target cell of interest.

Recombinant lentivirus capable of infecting a non-dividing cell wherein a suitable host cell is transfected with two or more vectors carrying the packaging functions, namely gag, pol and env, as well as rev and tat is described in U.S. Pat. No. 5,994,136. This describes a first vector that can provide a nucleic acid encoding a viral gag and a pol gene and another vector that can provide a nucleic acid encoding a viral env to produce a packaging cell. Introducing a vector providing a heterologous gene into that packaging cell yields a producer cell which releases infectious viral particles carrying the foreign gene of interest. The env preferably is an amphotropic envelope protein which allows transduction of cells of human and other species.

### Vaccinia Virus Vectors

Vaccinia virus vectors have been used extensively because of the ease of their construction, relatively high levels of expression obtained, wide host range and large capacity for carrying DNA. Vaccinia contains a linear, double-stranded DNA genome of about 186 kb that exhibits a marked "A-T" preference. Inverted terminal repeats of about 10.5 kb flank the genome. The majority of essential genes appear to map within the central region, which is most highly conserved among poxviruses. Estimated open reading frames in vaccinia virus number from 150 to 200. Although both strands are coding, extensive overlap of reading frames is not common.

At least 25 kb can be inserted into the vaccinia virus genome. Prototypical vaccinia vectors contain transgenes inserted into the viral thymidine kinase gene via homologous recombination. Vectors are selected on the basis of a tk-phenotype. Inclusion of the untranslated leader sequence of encephalomyocarditis virus results in a level of expression that is higher than that of conventional vectors, with the transgenes accumulating at 10% or more of the infected cell's protein in 24 h.

### Polyoma Viruses Vectors

The empty capsids of papovaviruses, such as the mouse polyoma virus, have received attention as possible vectors for gene transfer. The use of empty polyoma was first described when polyoma DNA and purified empty capsids were incubated in a cell-free system. The DNA of the new particle was protected from the action of pancreatic DNase. The reconstituted particles were used for transferring a transforming polyoma DNA fragment to rat FIII cells. The empty capsids and reconstituted particles consist of all three of the polyoma capsid antigens VP1, VP2 and VP3. U.S. Pat. No. 6,046,173 discloses the use of a pseudocapsid formed from papovavirus major capsid antigen and excluding minor capsid antigens, which incorporates exogenous material for gene transfer.

### Other Viral Vectors

Other viral vectors may be employed as expression constructs in the present invention, such as vectors derived from viruses such as sindbis virus or cytomegalovirus. They offer several attractive features for various mammalian cells (see e.g., Friedmann, Science, 1989; 244:1275-1281; Horwich et al., J. Virol., 1990; 64:642-650).

With the recognition of defective hepatitis B viruses, new insight was gained into the structure-function relationship of different viral sequences. *In vitro* studies showed that the virus could retain the ability for helper-dependent packaging and reverse transcription despite the deletion of up to 80% of its genome (Horwich et al., J. Virol. 64:642-650 (1990)). This suggested that large portions of the genome could be replaced with foreign genetic material. Chang *et al.* introduced the chloramphenicol acetyltransferase (CAT) gene into duck hepatitis R virus genome in the place of the polymerase, surface, and/or pre-surface coding sequences. It was cotransfected with wild-type virus into an avian hepatoma cell line. Culture media containing high titers of the recombinant virus were used to infect primary duckling hepatocytes. Stable CAT gene expression was detected for at least 24 days after transfection (Chang et al., Hepatology 14:134A (1991)).

### Modified Viruses

In still further embodiments of the present invention, the nucleic acids to be delivered are housed within an infective virus that has been engineered to express a specific binding ligand. The virus particle will thus bind specifically to the cognate receptors of the target cell and deliver the contents to the cell. A novel approach designed to allow specific targeting of retrovirus vectors was developed based on the chemical modification of a retrovirus by the chemical addition of lactose residues to the viral envelope. This modification can permit the specific infection of hepatocytes via sialoglycoprotein receptors.

Another approach to targeting of recombinant retroviruses was designed in which biotinylated antibodies against a retroviral envelope protein or against a specific cell receptor were used. The antibodies were coupled via the biotin components by using streptavidin. Using antibodies against major histocompatibility complex class I and/or class II antigens, they demonstrated the infection of a variety of human cells that bore those surface antigens with an ecotropic virus *in vitro.*

### Non-viral Transfer

DNA constructs of the present invention are generally delivered to a cell. In certain embodiments, however, the nucleic acid to be transferred is non-infectious, and can be transferred using non-viral methods.

Several non-viral methods for the transfer of expression constructs into cultured mammalian cells are contemplated by the present invention. Suitable methods for nucleic acid delivery for use with the current invention include methods as described herein or as would be known to one of ordinary skill in the art. Such methods include, but are not limited to, direct delivery of "naked" DNA plasmid via the vasculature (U.S. Pat. No. 6,867,196); by liposome mediated transfection (Nicolau and Sene, 1982; Fraley *et al.,* 1979; Nicolau *et al.,* 1987; Wong *et al.,* 1980; Kaneda *et al.,* 1989; Kato *et al.,* 1991) and receptor-mediated transfection (Wu and Wu, 1987; Wu and Wu, 1988); by agitation with silicon carbide fibers (Kaeppler *et al.,* 1990; U.S. Pat. Nos. 5,302,523 and 5,464,765); use of cationic lipids; naked DNA; or by microencapsulated DNA (U.S. Pat. Appl. No. 2005/0037085). Through the application of techniques such as these, target cells or tissue can be stably or transiently transformed.

Once the construct has been delivered into the cell, the nucleic acid encoding the therapeutic gene may be positioned and expressed at different sites. In certain embodiments, the nucleic acid encoding the therapeutic gene may be stably integrated into the genome of the cell. This integration may be in the cognate location and orientation via homologous recombination (gene replacement) or it may be integrated in a random, non-specific location (gene augmentation). In yet further embodiments, the nucleic acid may be stably maintained in the cell as a separate, episomal segment of DNA. Such nucleic acid segments or "episomes" encode sequences sufficient to permit maintenance and replication independent of or in synchronization with the host cell cycle. How the expression construct is delivered to a cell and where in the cell the nucleic acid remains is dependent on the type of expression construct employed.

In a particular embodiment of the invention, the expression construct may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers. The addition of DNA to cationic liposomes causes a topological transition from liposomes to optically birefringent liquid-crystalline condensed globules. These DNA-lipid complexes are potential non-viral vectors for use in gene therapy.

Liposome-mediated nucleic acid delivery and expression of foreign DNA *in vitro* has been very successful. Using the β-lactamase gene, investigators demonstrated the feasibility of liposome-mediated delivery and expression of foreign DNA in cultured chick embryo, HeLa, and hepatoma cells. Successful liposome-mediated gene transfer in rats after intravenous injection has also been accomplished. Also included are various commercial approaches involving "lipofection" technology.

In certain embodiments of the invention, the liposome may be complexed with a hemagglutinating virus (HVJ). This has been shown to facilitate fusion with the cell membrane and promote cell entry of liposome-encapsulated DNA. In other embodiments, the liposome may be complexed or employed in conjunction with nuclear non-histone chromosomal proteins (HMG-1). In yet further embodiments, the liposome may be complexed or employed in conjunction with both HVJ and HMG-I. In that such expression constructs have been successfully employed in transfer and expression of nucleic acid *in vitro* and *in vivo,* then they are applicable for the present invention.

Other vector delivery systems which can be employed to deliver a nucleic acid encoding a therapeutic gene into cells are receptor-mediated delivery vehicles. These take advantage of the selective uptake of macromolecules by receptor-mediated endocytosis in almost all eukaryotic cells. Because of the cell type-specific distribution of various receptors, the delivery can be highly specific (Wu and Wu, 1993). Where liposomes are employed, other proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, and proteins that target intracellular localization and enhance intracellular half-life.

Receptor-mediated gene targeting vehicles generally consist of two components: a cell receptor-specific ligand and a DNA-binding agent. Several ligands have been used for receptor-mediated gene transfer. The most extensively characterized ligands are asialoorosomucoid (ASOR) and transferring (Wagner et al., Proc. Natl. Acad. Sci. 87(9):3410-14 (1990)). A synthetic neoglycoprotein, which recognizes the same receptor as ASOR, has been used as a gene delivery vehicle. Epidermal growth factor (EGF) has also been used to deliver genes to squamous carcinoma cells.

In other embodiments, the delivery vehicle may comprise a ligand and a liposome. For example, investigators have employed lactosyl-ceramide, a galactose-terminal asialganglioside, incorporated into liposomes and observed an increase in the uptake of the insulin gene by hepatocytes. Thus, it is feasible that a nucleic acid encoding a therapeutic gene also may be specifically delivered into a cell type such as cardiac cells, by any number of receptor-ligand systems with or without liposomes.

In another embodiment of the invention, the expression construct may simply consist of naked recombinant DNA or plasmids. Transfer of the construct may be performed by any of the methods mentioned above which physically or chemically permeabilize the cell membrane. This is applicable particularly for transfer *in vitro,* however, it may be applied for *in vivo* use as well. It is envisioned that therapeutic DNA may also be transferred in a similar manner *in vivo.* Wolff et al. (U.S. Pat. No. 6,867,196) teach that efficient gene transfer into heart tissue can be obtained by injection of plasmid DNA solutions in a vein or artery of the heart. Wolff also teaches the administration of RNA, non-plasmid DNA, and viral vectors.

The vectors useful in the present invention have varying transduction efficiencies. As a result, the viral or non-viral vector transduces more than, equal to, or at least about 10%, 20%, 30%, 40%, 50%, 55%, 60%, 65%, 70%, 75%, 80%, 85%, 90%, 95%, 99%, 100% of the cells of the targeted vascular territory. More than one vector (viral or non-viral, or combination thereof) can be used simultaneously, or in sequence. This can be used to transfer more than one polynucleotide, and/or target more than one type of cell. Where multiple vectors or multiple agents are used, more than one transduction/transfection efficiency can result.

### Therapeutic Substances

The therapeutic substances, including polynucleotides, that are useful in the present invention are utilized to treat cardiovascular disease. These substances include compounds known to treat any aspect of cardiovascular disease. The polynucleotide can target any of the known nucleic acids or proteins of the cardiovascular system resulting in modulating a cellular activity of the heart tissue. Of particular interest are the nucleic acids and proteins required for contraction of heart muscle, including the nucleic acids and proteins which regulate calcium concentrations in heart muscle.

Each muscle contraction requires the entry of Ca²⁺ into the muscle cell cytoplasm, while relaxation requires removal of the activating Ca²⁺, making Ca²⁺ regulation of contraction one of the most extensive activities in the human body. It is not surprising, therefore, that proteins involved in Ca²⁺ regulation, when mutated, can lead to a variety of skeletal and cardiac muscle diseases that are related to defects in Ca²⁺ regulation.

Sarcoplasmic reticulum calcium-ATPases (SERCA) pump calcium from the cytoplasm of mammalian cells into organellar structures such as the sarcoplasmic reticulum in muscle or the endoplasmic reticulum in non-muscle cells. Their threshold of activation by calcium is of the order of 100-200 nM, so that they set the resting level of cytoplasmic calcium. Abnormal calcium cycling, characteristic of experimental and human heart failure, is associated with impaired sarcoplasmic reticulum calcium uptake activity

SERCA2a, the cardiac/slow-twitch isoform binds to and is regulated by phospholamban (PLN), a 52 amino acid, homopentameric protein made up of three domains. During muscle contraction, phospholamban inhibits the Ca²⁺ pump. During muscle relaxation, it can be phosphorylated, which removes the inhibition and allows the Ca²⁺ to be pumped back into sarcoplasmic reticulum. The regulation is thought to be primarily from a physical interaction between the phospholamban monomer and the pump. However, the 52 residue peptide also associates into pentamers which have been shown to be selective for Ca²⁺ ions.

Impaired sarcoplasmic reticulum calcium uptake activity reflects decreases in the cAMP-pathway signaling and increases in type 1 phosphatase activity. The increased protein phosphatase 1 activity is partially due to dephosphorylation and inactivation of its inhibitor-1, promoting dephosphorylation of phospholamban and inhibition of the sarcoplasmic reticulum calcium-pump. Indeed, cardiac-specific expression of a constitutively active inhibitor-1 results in selective enhancement of phospholamban phosphorylation and augmented cardiac contractility at the cellular and intact animal levels. Notably, acute adenoviral gene delivery of the active inhibitor-1 completely restores function and partially reverses remodeling, including normalization of the hyperactivated p38, in the setting of pre-existing heart failure (Pathak et al. Circ Res., 2005; 96(7):756-66 ).

Ventricular arrhythmias can cause sudden cardiac death (SCD) in patients with normal hearts and in those with underlying disease such as heart failure. In animals with heart failure and in patients with inherited forms of exercise induced SCD, depletion of the channel-stabilizing protein calstabin2 (FKBP12.6) from the ryanodine receptor-calcium release channel (RyR2) complex causes an intracellular Ca²⁺ leak that can trigger fatal cardiac arrhythmias. Increased levels of calstabin2 stabilize the closed state of RyR2 and prevent the Ca²⁺ leak that triggers arrhythmias. Thus, enhancing the binding of calstabin2 to RyR2 is thought to be a therapeutic strategy for common ventricular arrhythmias.

The nucleic acids and proteins of SERCA, phospholamban, inhibitor-1 of the type 1 phosphatase, S100A1, and sarcolipin, as well as related nucleic acids and proteins which play a role in Ca²⁺, are preferred targets for polynucleotides of the present invention.

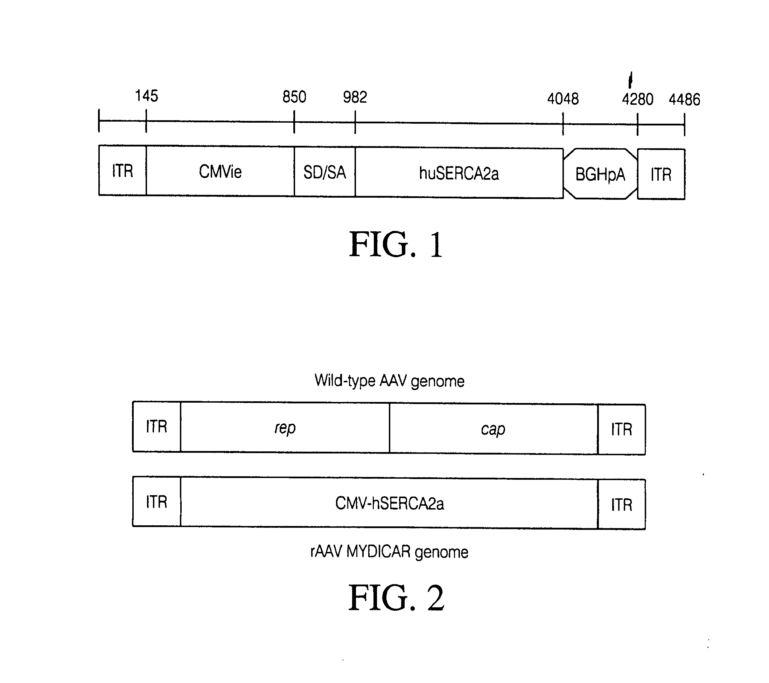

A preferred viral vector and transgene is AAV2/1/SERCA2a, which is comprised of an AAV serotype 1 viral capsid enclosing a single-stranded 4486 nucleotide DNA containing the human SERCA2a expression cassette flanked by ITRs derived from AAV serotype 2. The icosahedral capsid consists of three related AAV serotype 1 capsid proteins, VP1, VP2, and VP3. The AAV2/1/SERCA2a DNA contains the following components: AAV serotype2 based ITR at the 3' and 5' ends, flanking the CMV-hSERCA2a-polyA expression cassette. The expression cassette contains the cytomegalovirus immediate early enhancer/promoter (CMVie) driving transcription of sequences including a hybrid intron from the commercial plasmid pCI (Promega - GenBank U47119), the hSERCA2A cDNA (coding sequence identical to GenBank NM-001681), and a bovine growth hormone polyadenylation signal [BGHpA, (GenBank M57764)]. The hybrid intron was designed using the 5'-donor site from the first intron of the human b-globin and 3'-acceptor site from the intron located between the leader and body of an immunoglobulin gene heavy chain variable region (see Figure 1).

The AAV2/1/SERCA2a vector incorporates less than 300 nucleotides of the wild-type AAV (wtAAV) sequences in the vector genome. The wtAAV sequences are AAV serotype 2 derived ITRs that provide in *cis* the packaging signal (Figure 2) that allows the SERCA2a DNA to be inserted into the capsid.

Without being tied to a particular mechanism of action, it is believed that SERCA2a protein levels are decreased in the cardiomyocytes of patients with CHF, and that increasing the levels of this protein in cardiomyocytes will normalize the abnormally high diastolic levels of cytosolic calcium typical of CHF and improve clinical outcomes.

The therapeutic agents, including polynucleotides, polynucleotides in combination with a vector, both viral and non-viral, as discussed above can be used in the preparation of a medicament for the treatment of cardiovascular disease, where the medicament is administered by direct infusion into the coronary circulation.

### Therapeutic Effect

In a preferred embodiment, the infusion of the therapeutic agents disclosed herein are used to achieve a therapeutic effect in a patient suffering from cardiac disease. The treated individual may be monitored for clinical features which accompany the cardiac disorder. For example, subjects may be monitored for reduction in adverse signs and symptoms associated with cardiovascular disease. For example, after treatment of congestive heart failure in a subject using methods of the present invention, the subject may be assessed for improvements in a number of clinical parameters including, but not limited to, increased lateral ventricle fractional shortening; augmented cardiac contractility at the cellular and intact animal levels, reversal of cardiac remodeling, and normalization of the abnormally high diastolic levels of cytosolic calcium. Other clinical features which can be monitored in a subject treated with the present invention include without limitation survival, cardiac metabolism, heart contractility, heart rate, ventricular function (e.g., left ventricular end-diastolic pressure (LVEDP), left ventricular systolic pressure (LVSP)), Ca²⁺ metabolism (e.g., intracellular Ca²⁺ concentration, peak or resting [Ca²⁺], SR Ca²⁺ ATPase activity, phosphorylation state of phospholamban), force generation, relaxation and pressure of the heart, a force frequency relationship, cardiocyte survival or apoptosis or ion channel activity (e.g., sodium calcium exchange, sodium channel activity, calcium channel activity, sodium potassium ATPase pump activity), activity of myosin heavy chain, troponin I, troponin C, troponin T, tropomyosin, actin, myosin light chain kinase, myosin light chain 1, myosin light chain 2 or myosin light chain 3, IGF-1 receptor, PI3 kinase, AKT kinase, sodium-calcium exchanger, calcium channel (L and T), calsequestrin, or calreticulin. The evaluation can be performed before, after, or during the treatment. Other measures of cardiac disease which can be monitored include fractional shortening, cardiac output, ejection fraction, Tau, regurgitant volume, number of hospital stays, quality of life, and treadmill time.

The disclosed methods and therapeutic agents disclosed herein can be combined with existing treatments for cardiac disease, such as drugs or surgical intervention, to provide an enhanced therapeutic effect compared to existing treatments alone. An enhanced therapeutic effect may be demonstrated by, for example, an extension of the time period between the worsening of the signs or symptoms of the disease compared to the average or typical time period for existing treatment regimens, or the lengthening of time required before additional treatment is required compared to the average or typical time for standard treatment alone.

Embodiments of the invention will now be further described in the following non-limiting examples.

### Example 1: Effect of Infusion Time on Short Term Biodistribution of AAV2/1/SERCA2a in Normal Minipigs

A study was conducted to evaluate transduction of heart muscle by quantification of vector-specific DNA after antegrade epicardial coronary infusion of AAV2/1/SERCA2a in normal minipigs.

### Groups

The control group (one animal) for this study was a no treatment control. This animal underwent all the same procedures as the other groups except for administration of AAV2/1/SERCA2a. Experimental animals were administered 1 x 10¹² DRP AAV2/1/SERCA2a via coronary artery infusion catheter using a programmable syringe pump into the left coronary artery (5 animals in Groups 3-5) or direct intramuscular (IM) injection (a single animal in Group 2). Groups received AAV2/1/SERCA2a at two vector concentrations and varying infusion rates. All groups receiving vector received a blood only flush of the dead volume of the tubing and catheter following vector administration. The animals were Gottingen Minipig, at least four months old, weighing between 8-12 kg. Animals were assigned to one of five groups, as shown in Table 1.

**Table 1: Group Designation**

| **Treatment Group Concentration/Infusion Time** | **Total Dose (DRP)** | **No./ Group** | **Vector Infusion Time (min)** |
|---|---|---|---|
| ***Group 1:*** No treatment control | N/A | 1 | N/A |
| ***Group 2:*** Direct IM injection | 1 x 10¹² | 1 | N/A |
| ***Group 3:*** Bolus injection (-0.5 minute) | 1 x 10¹² | 1 | 0.5 |
| ***Group 4:*** 10 minute infusion | 1 x 10¹² | 3 | 10 |
| ***Group 5:*** 15 minute infusion | 1 x 10¹² | 1 | 15 |

### Anesthesia / Blood Sample

On Day 0, animals were anesthetized using standard procedures. An antibiotic was administered prophylactically into the muscle of a hind leg. Vital signs (heart rate, respiratory rate, and O₂ pulse oximeter) were monitored. An ECG was obtained for monitoring purposes.

### AAV2/1/SERCA2a Administration

### AAV2/1/SERCA2a Administration Direct IM Injection: Group 2

On Day 0, a standard intramuscular syringe and needle was used to inject AAV2/1/SERCA2a into the rear muscle quadrant. The volume and concentration administered are provided in Table 2 below. AAV2/1/SERCA2a was stored frozen at -70 ± 10°C or below until use. On the day of use, the AAV2/1/SERCA2a was thawed at room temperature and held on ice until the animal was ready for dosing. After gentle mixing by inversion, 0.72 mL of AAV2/1/SERCA2a stock solution at 1.4 x 10¹² DRP/mL was aseptically transferred into a standard intramuscular syringe and needle. The single administration of a total dose of 1 x 10¹² DRP AAV2/1/SERCA2a was then injected intramuscularly in the rear muscle quadrant.

### General Procedures: AAV2/1/SERCA2a Administration by Antegrade Epicardial Coronary Infusion (Groups 3-5)

The direct infusion system is composed of standard (commercially available) components including a conventional guide sheath, 0.014" guide wire, a 5F infusion (guide) catheter and two programmable syringe pumps. An important aspect of this method is the infusion times used for vector administration. Exact volumes can vary based on starting vector concentrations, dead volumes of tubing and catheter units, etc.

On Day 0, the direct infusion procedure began with introduction of the guide sheath using a femoral arterial approach. A coronary infusion catheter (e.g., Cordis Vista Brite Tip Guiding Catheter or similar model appropriate for cannulation of the left main coronary artery) was then placed in the left main coronary artery under fluoroscopic guidance. Once the catheter was in place, it was connected to the first programmable syringe pump (e.g., NE-1000 Programmable Syringe Pump, New Era Pump Systems) using standard tubing and purging technique. The AAV2/1/SERCA2a was then delivered, followed by a blood-only flush of the catheter dead volume with a second programmable syringe pump. A detailed list of required materials and procedural steps for the direct infusion method is provided in Tables 3 and 4. Dilution volumes, infusion times, infusion rates, volume delivered prior to blood only flush, and times needed to complete the blood only flush are provided in Table 2.

### Group 3: AAV2/1/SERCA2a Administration (Bolus Injection)

After gentle mixing by inversion, 0.72 mL of AAV2/1/SERCA2a stock solution at 1.4 x 10¹² DRP/mL and 1.3 mL of Formulation Buffer (130 mM NaCl, 20 mM HEPES and 1 mM MgCl₂ at pH 7.4) were aseptically transferred into a sterile polypropylene vessel resulting in a concentration of 5.0 x 10¹¹ DRP/mL. Just prior to dosing the animal, 2.0 mL of the diluted AAV2/1/SERCA2a (5 x 10¹¹ DRP/mL) was brought to room temperature and mixed with 1.0 mL of whole native blood from the animal being dosed, resulting in a final dilution in the syringe of 3.3 x 10¹¹ DRP/mL in a final volume of 3.0 mL. The circuit was primed with blood and then the vector was injected into the left main coronary artery over a 0.5 minute period. This was followed by blood-only flush of the catheter dead volume.

### Group 4: AAV2/1/SERCA2a Administration (10 Minute Vector Infusion)

After gentle mixing by inversion, 0.72 mL of AAV2/1/SERCA2a stock solution at 1.4 x 10¹² DRP/mL and 7.3 mL Formulation Buffer were aseptically transferred into a sterile polypropylene vessel resulting in a concentration of 1.25 x 10¹¹ DRP/mL. Just prior to dosing the animal, 8.0 mL of diluted AAV2/1/SERCA2a (1.25 x 10¹¹ DRP/mL) was brought to room temperature and mixed with 4.0 mL of whole native blood from the animal being dosed. The infusion circuit was primed with blood and then the vector was delivered into the left main coronary artery over a 10 minute period using a programmable syringe pump, followed by blood-only flush of the catheter dead volume with a second programmable syringe pump.

### Group 5: AAV2/1/SERCA2a Administration (15 Minute Vector Infusion)

After gentle mixing by inversion, 0.72 mL of AAV2/1/SERCA2a stock solution at 1.4 x 10¹² DRP/mL and 1.3 mL of Formulation Buffer were aseptically transferred into a sterile polypropylene vessel resulting in a concentration of 5.0 x 10¹¹ DRP/mL. Just prior to dosing the animal, 2.0 mL of diluted AAV2/1/SERCA2a (5 x 10¹¹ DRP/mL) was brought to room temperature and mixed with 1.0 mL of whole native blood from the animal being dosed. The infusion circuit was primed with blood and then the vector was delivered into the left main coronary artery over a 15 minute period using a programmable syringe pump, followed by blood-only flush of the catheter dead volume with a second programmable syringe pump.

**Table 2: Dilution/Administration Scheme**

| **Treatment Group Concentration/Infusion Time** | **Total Dose (DRP)** | **Volume Diluted Vector (mL)** | **Volume of Blood (mL)** | **Total Volume of Vector + Blood Administered (mL)** | **Vector Infusion Time (min)** | **Infusion Rate (mL/min)** | **Time for Blood-Only Flush (min)** |
|---|---|---|---|---|---|---|---|
| ***Group 1:*** No treatment control | N/A | N/A | N/A | N/A | N/A | N/A | N/A |
| ***Group* 2:** Direct IM injection | 1 x 10¹² | 0 | 0 | 0.72 | N/A | N/A | N/A |
| ***Group* 3:** Bolus injection (-0.5 minute) | 1 x 10¹² | 2.0 | 1.0 | 3.0 | 0.5 | 6.0 | I |
| ***Group* 4:** 10 minute infusion | 1 x 10¹² | 8.0 | 4.0 | 12.0 | 10 | 1.2 | 2 |
| ***Group 5:*** 15 minute infusion | 1 x 10¹² | 2.0 | 1.0 | 3.0 | 15 | 0.2 | 10 |

**Table 3: Materials for Direct Infusion Method**

| **Quantity** | **Material** |
|---|---|
| 1 | A standard guiding catheter (5F) with appropriate shape |
| As necessary | Any hemostatic valve, 3-way stopcock or any other accessories used in interventional cardiac procedures |
| 1 | 0.014" x 175 cm (minimum length) guide wire (optional) |
| 1 | Guide wire introducer (optional) |
| 2 | Programmable syringe pump (NE-1000 Programmable Syringe Pump, New Era Pump Systems) |
| 2-3 | 10-20 cc syringes |

**Table 4: Procedural Steps for Direct Infusion Method**

| **Step** | **Procedure** |
|---|---|
| I | Prior to using the direct infusion system, carefully remove the components from their packages and inspect for bends, kinks, and other damage. Do not use if any defects are noted. |
| 2 | Prepare arterial access site according to standard interventional practice. |
| 3 | Obtain arterial access via placement of a standard introducer sheath. |
| 4 | Under fluoroscopic guidance and following standard interventional cardiac procedures, introduce a standard guide catheter into the left main coronary artery (use a catheter shape that is appropriate for the specific vascular anatomy) |
| 5 | Administer heparin according to institutional procedures. |
| 6 | Prepare first programmable syringe pump for delivery of AAV2/1/SERCA2a. |
| 7 | Prime tubing with native blood from the animal being dosed and purge all air from the tubing. |
| 8 | Connect the distal end of the tubing to the hemostasis valve on the guide (infusion) catheter. Insure that all air is removed from the tubing. |
| 9 | Connect the proximal end of the tubing to the AAV2/1/1/SERCA2a loaded syringe |
| 10 | Position the AAV2/1/SERCA2a loaded syringe into the syringe pump |
| 11 | Start the first pre-programmed pump and run it for the amount of time specified in the protocol |
| 12 | Prepare a second programmable syringe pump and prime tubing with native blood from the animal being dosed and purge all air from the tubing. |
| 13 | Deliver the AAV2/1/SERCA2a at the flow rate specified in the study protocol |
| 14 | Upon completion of the delivery period, stop the first syringe pump and disconnect the proximal end of the tubing. |
| 15 | Immediately connect the proximal end of the tubing to the second syringe pump filled with native blood from the animal being dosed. |
| 16 | Restart the second syringe pump (at the same flow rate used to deliver the AAV2/1/SERCA2a) and infuse the blood. |
| 17 | Upon completion of the catheter dead volume flush, stop the syringe pump. |
| 18 | Turn the stopcock on the hemostasis valve to the off position and disconnect the tubing. |
| 19 | Remove the guide catheter (infusion) following standard procedures. |
| 20 | Dispose of the tubing and syringes following institutional procedures. |

### Day 2: Terminal Sacrifice and PCR Tissue Samples

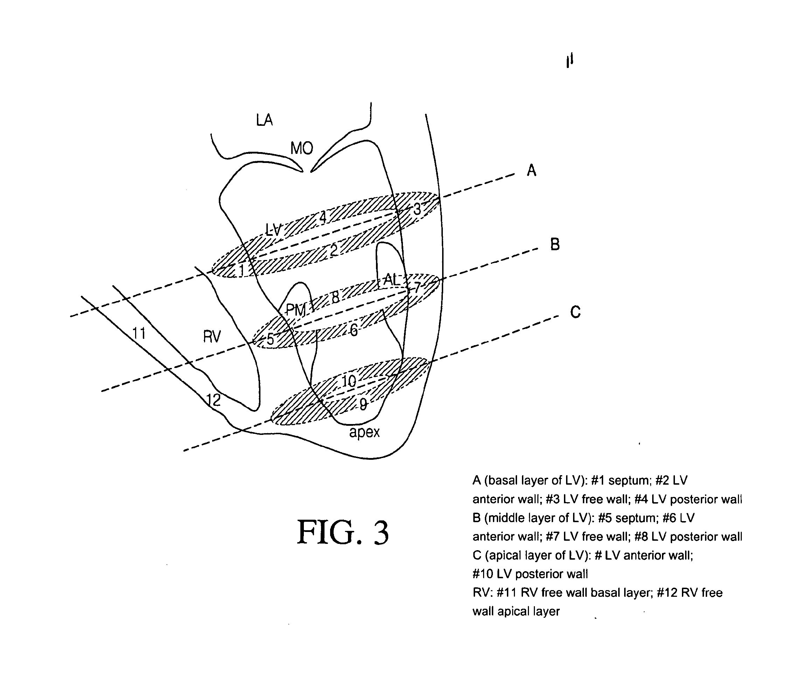

Blood samples were collected for clinical chemistry following administration of anesthesia. Animals were then euthanized by intravenous administration of 30 mg/kg potassium chloride and PCR tissue samples collected, using standard techniques, from the following areas of the heart as illustrated in Figure 3: anterior wall, posterior wall, septum and free wall in the basal and middle layers of the left ventricle; anterior wall and posterior wall in the apical layer of the left ventricle; and the free wall from the basal layer and apical layers of the right ventricle.

### PCR Analysis of Samples

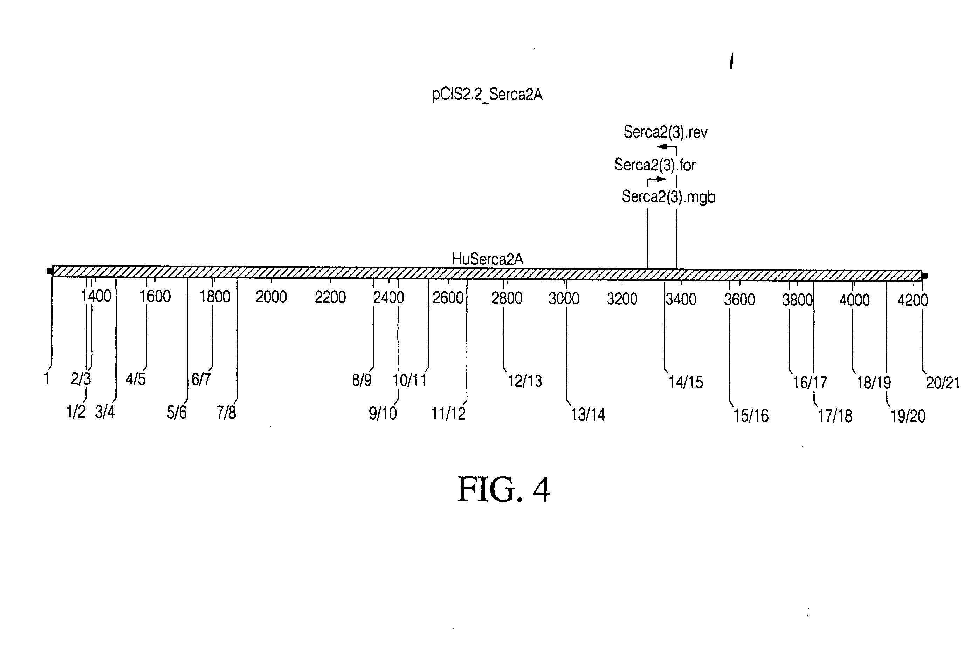

A quantitative polymerase chain reaction (qPCR) assay was used to detect and quantify AAV2/1/SERCA2a in tissue samples collected. The qPCR assay detects a 107 base pair sequence unique to AAV2/1/SERCA2a using the ABI Prism 7700 Sequence Detection System. The qPCR primers span exons 14 and 15 as indicated in the diagram of Figure 4. The number of copies of AAV2/1/SERCA2a detected in one microgram of genomic DNA extracted from each tissue sample was quantified using serial dilutions of a plasmid containing the target sequences (pcDNA3.1_huSERCA2) as standards. The lower limit of detection of the assay was 20 copies of AAV2/1/SERCA2a/µg DNA; the lower limit of quantification was 200 copies/µg DNA.

### Results

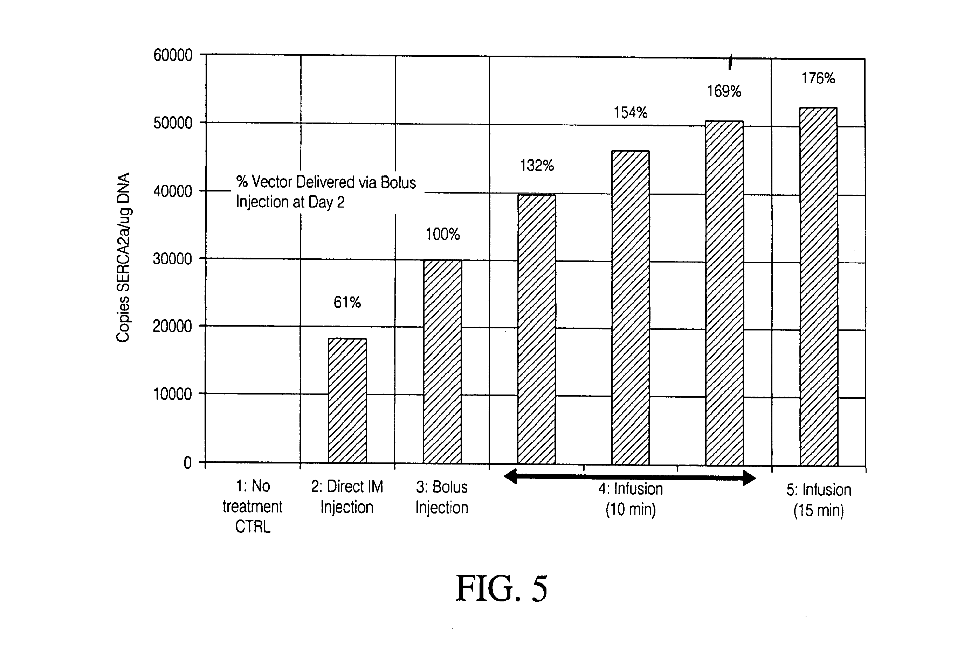

The results of the experiment are depicted in Figure 5, where the copies of AAV2/1/SERCA2a/µg DNA are reported, along with the relative amount expressed as a percentage of the level achieved after bolus injection (30 sec, Group 3). The values from the twelve different heart samples (Figure 3) were averaged into a single value for each animal, and the values are reported on a per animal basis. Compared to a bolus injection (0.5 min.), an infusion time of 10 minutes resulted in an average of 51.6% higher copies (32%-69%), and an infusion time of 15 minutes resulted in 76% more copies than the bolus injection. Applicants note that while direct IM injection resulted a substantial number of copies, this is a result of animals being sacrificed on Day 2 and the ongoing clearance of AAV vector by reticulo-endothelial system (monocyte/macrophage) (Brain J.D., et al., Am J Physiol. Lung Cell Mol. Physiol., 1999; 276:146-154). IM injection of AAV1 vectors does not result in significant long-term transduction of the heart (Rip J, et al., Hum. Gene Ther., 2005; 16(11):1276-86). In contrast, antegrade epicardial coronary infusion results in stable transduction of the tissue as demonstrated in Example 3. These results demonstrate that extended infusion times result in more copies of AAV2/1/SERCA2a per µg DNA, even though the same amount of AAV2/1/SERCA2a (1 x 10¹² DRP) was administered in each group.

### Example 2: Effect of Vector Dose and Administration Route on Short Term Biodistribution of AAV2/1/SERCA2a in Sheep

A pilot study was conducted to evaluate the short term biodistribution of three different doses of AAV2/1/SERCA2a at 2 days following a single administration via infusion into the left coronary artery compared to an intravenous injection in normal sheep.

### Groups

One animal received 1 x 10¹³ DRP of AAV2/1/SERCA2a by an intracoronary artery catheter infused into the left coronary artery using a programmable syringe pump. A second group of three animals received 3 x 10¹² DRP of AAV2/1/SERCA2a, and a third group of two animals received 1 x 10¹² DRP of AAV2/1/SERCA2a, all by an intracoronary artery catheter infused into the left coronary artery using a programmable syringe pump. All infusion groups received the AAV2/1/SERCA2a over 8 minutes at a constant flow rate of 2.5 mL/min., followed by a 2 minute blood-only flush of the catheter volume. A single animal in a control group was administered 1 x 10¹² DRP AAV2/1/SERCA2a in an intravenous injection of 2 mL. The groups are shown below in Table 5 and the administration schedule is shown in Table 6.

**Table 5: Group Designations**

| **TREATMENT GROUP** | | **TOTAL** | **DOSE** |
|---|---|---|---|
| ***GROUP 1—*** | *AAV2*/*1*/*SERCA2A VIA INFUSION BY SYRINGE PUMP INTO THE LEFT CORONARY ARTERY* | 3 | 1 x 10¹³ DRP |
| ***GROUP 2—*** | *AAV2*/*1lSERCA2A VIA INFUSION BY SYRINGE PUMP INTO THE LEFT CORONARY ARTERY* | 3 | 3 x 10¹² DRP |
| ***GROUP 3—*** | *AAV2*/*1*/*SERCA2A VIA INFUSION BY SYRlNGE PUMP INTO THE LEFT CORONARY ARTERY* | 2 | 1 x 10¹² DRP |
| ***GROUP 4—*** | *AAV2*/*1*/*SERCA2A VIA INTRAVENOUS INJECTION* | 1 | 1 x 10¹² DRP |

**Table 6: Dilution/Administration Scheme**

| **Treatment Group Concentration/ Administration Route** | **Total Dose (DRP)** | **Volume Diluted Vector (mL)** | **Volume of Blood (mL)** | **Total Volume of Vector + Blood Administered (mL)** | **Vector Infusion / Injection Time (min)** | **Infusion Rate (mL/min)** | **Time for Blood-Only Flush (min)** |
|---|---|---|---|---|---|---|---|
| ***Group 1:*** 1 x 10¹³ DRP intracoronary infusion | 1 x 10¹³ | 10 | 10 | 20 | 8 | 2.5 | 2 |
| ***Group 2:*** 3 x 10¹² DRP intracoronary infusion | 3 x 10¹² | 10 | 10 | 20 | 8 | 2.5 | 2 |
| ***Group 3:*** 1 x 10¹² DRP intracoronary infusion | 1 x 10¹² | 10 | 10 | 20 | 8 | 2.5 | 2 |
| ***Group 4:*** 1 x 10¹² DRP i.v. injection | 1 x 10¹² | 2.0 | N/A | N/A | ~0.5 | N/A | N/A |

### AAV2/1/SERCA2a Administration

### General Procedures: AAV2/1/SERCA2a Administration by Antegrade Epicardial Coronary Infusion (Groups 1-3)

On Day 0, all animals were administered a full dose of heparin to achieve an ACT>300. A coronary artery infusion catheter was used to infuse AAV2/1/SERCA2a into the left coronary artery using a programmable syringe pump. The materials and procedures for implanting the catheter are described in detail in Example 1 and Tables 3 and 4 above. Essentially the same procedure was followed.

### Group 1: 1.0 x 10¹³ AAV2/1/SERCA2a Administration (8 Minute Vector Infusion)

After gentle mixing by inversion, AAV2/1/SERCA2a stock solution was aseptically transferred to a sterile polypropylene tube and diluted with Formulation Buffer (130 mM NaCl, 20 mM HEPES and 1 mM MgCl₂ at pH 7.4) to a total volume of 10 mL, resulting in a concentration of 1 x 10¹² DRP/mL. Just prior to dosing the animal, 10 mL of diluted AAV2/1/SERCA2a (1 x 10¹² DRP/mL) was brought to room temperature and mixed with 10 mL of whole native blood from the animal being dosed, resulting in a final volume of 20.0 mL. The infusion circuit was primed with blood and then the vector was delivered into the left main coronary artery over an 8 minute period at a constant rate of 2.5 mL/min. using a programmable syringe pump, followed by blood-only flush of the catheter dead volume with a second programmable syringe pump.

### Group 2: 3.0 x 10¹² AAV2/1/SERCA2a Administration (8 Minute Vector Infusion)

After gentle mixing by inversion, AAV2/1/SERCA2a stock solution was aseptically transferred to a sterile polypropylene tube and diluted with Formulation Buffer (130 mM NaCl, 20 mM HEPES and 1 mM MgCl₂ at pH 7.4) to a total volume of 10 mL, resulting in a concentration of 3 x 10¹¹ DRP/mL. Just prior to dosing the animal, 10 mL of diluted AAV2/1/SERCA2a (3 x 10¹¹ DRP/mL) was brought to room temperature and mixed with 10 mL of whole native blood from the animal being dosed, resulting in a final volume of 20.0 mL. The infusion circuit was primed with blood and then the vector was delivered into the left main coronary artery over an 8 minute period at a constant rate of 2.5 mL/min. using a programmable syringe pump, followed by blood-only flush of the catheter dead volume with a second programmable syringe pump.

### Group 3: 1.0 x 10¹² AAV2/1/SERCA2a Administration (8 Minute Vector Infusion)

After gentle mixing by inversion, AAV2/1/SERCA2a stock solution was aseptically transferred to a sterile polypropylene tube and diluted with Formulation Buffer (130 mM NaCl, 20 mM HEPES and 1 mM MgCl₂ at pH 7.4) to a total volume of 10 mL, resulting in a concentration of 1 x 10¹¹ DRP/mL. Just prior to dosing the animal, 10 mL of diluted AAV2/1/SERCA2a (1 x 10¹¹ DRP/mL) was brought to room temperature and mixed with 10 mL of whole native blood from the animal being dosed, resulting in a final dilution in the syringe of 0.5 x 10¹¹ DRP/mL in a final volume of 20.0 mL. The infusion circuit was primed with blood and then the vector was delivered into the left main coronary artery over an 8 minute period at a constant rate of 2.5 mL/min. using a programmable syringe pump, followed by blood-only flush of the catheter dead volume with a second programmable syringe pump.

### Group 4: 1.0 x 10¹² AAV2/1/SERCA2a Administration (I.V. Injection)

After gentle mixing by inversion, AAV2/1/SERCA2a stock solution was aseptically transferred to a sterile polypropylene tube and diluted with Formulation Buffer (130 mM NaCl, 20 mM HEPES and 1 mM MgCl₂ at pH 7.4) to a total volume of 2.0 mL, resulting in a concentration of 1 x 10¹² DRP/mL. The solution was administered by intravenous injection (~0.5 min.) using a standard i.v. syringe and needle.

### Day 2: Terminal Sacrifice and PCR Tissue Samples

Following administration of anesthesia, animals were euthanized by intravenous administration of 30 mg/kg potassium chloride and PCR tissue samples were collected using standard techniques. Samples were collected from the following areas of the heart: anterior wall of the left ventricle, inferior wall of the left ventricle, interventricular septum, and right ventricular free wall.

### PCR Analysis of Samples

The quantitative PCR assay described in Example 1 was used to detect and quantify AAV2/1/SERCA2a in tissue samples collected.

### Results

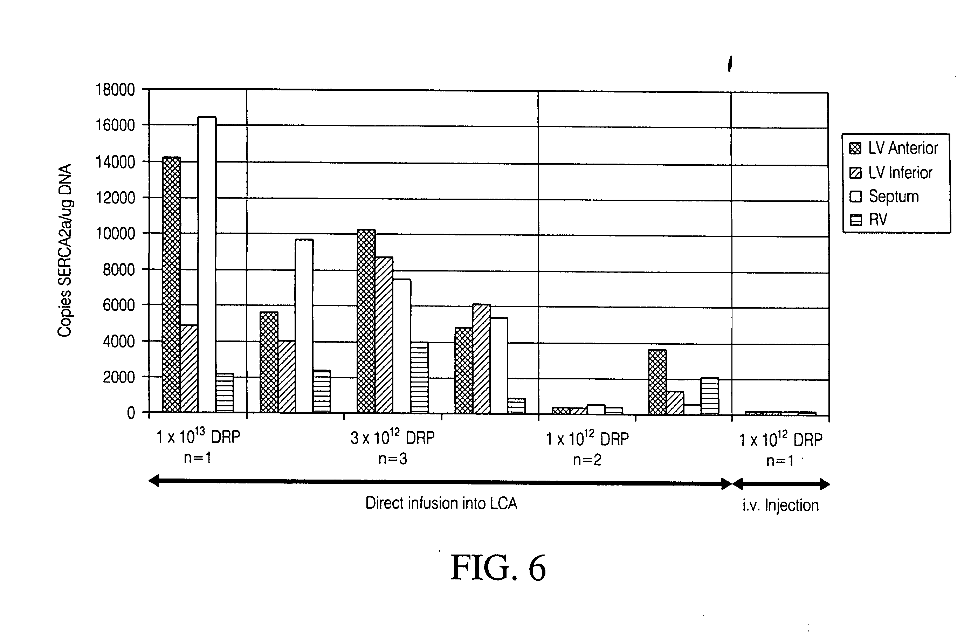

The results of the experiment are depicted in Figure 6, where the copies of AAV2/1/SERCA2a / µg DNA in each sample of heart tissue are reported for each animal. Unlike an intravenous injection (~0.5 min.) which resulted in very low, non-quantifiable levels (20-200 copies) of AAV2/1/SERCA2a, an infusion time of 8 minutes resulted in a significant number of copies at the 1 x 10¹², 3 x 10¹², and 1 x 10¹³ concentrations. Figure 6 shows that a total dose of 1 x 10¹³ DRP resulted in a greater number of copies than a total dose of 3 x 10¹² DRP, which in turn resulted in a greater number of copies than a total dose of 1 x 10¹² DRP. Importantly, copies of AAV2/1/SERCA2a were found in the right ventricle samples even though the vector was administered to only the left coronary artery.

### Example 3: Swine Mitral Valve Regurgitation Model of Heart Failure

A pilot study was conducted to evaluate the effect of a single administration of AAV2/1/SERCA2a on cardiac function as compared to a vehicle control in a porcine model of heart failure created by severe mitral valve regurgitation (MR). Mitral regurgitation (MR), also known as mitral insufficiency, is the abnormal leaking of blood through the mitral valve, from the left ventricle into the left atrium of the heart. Regurgitant volume, a measure of the severity of the MR, is the volume of blood that regurgitates into the left atrium

### Groups

Yorkshire-Landrace swine, 3 months old, and between 30-40 kg were used in the study. The experimental group of four animals received 1 x 10¹² DRP of AAV2/1/SERCA2a by an intracoronary artery catheter infused into the left coronary artery using a Harvard Clinical Technology (HCT) infusion pump. The AAV2/1/SERCA2a was delivered over a period of 8 minutes at a constant flow rate of 2.5 mL/min. Following completion of the infusion, the remaining solution was withdrawn from the tube connecting the end of the guiding catheter and the pump. This remaining solution was then infused manually over a period of approximately 2 minutes, followed by slow manual flush with 10 mL of saline. The control group was 5 animals which received Formulation buffer (130 mM NaCl, 20 mM HEPES and 1 mM MgCl₂ at pH 7.4) instead of AAV2/1/SERCA2a, either by direct infusion (DI) or by V-Focus device. Preovolos AC, et al., J. Extra Corpor. Technol. 2006; 38(1):51-2).

### ANIMAL PROCEDURES

### Day 0 - Creation of MR and Recovery

### Anesthesia, Intubation and Anticoagulation

Telazol 6.0 mg/kg IM and Atropine 2.0 mL IM was injected for the introduction of anesthesia. General anesthesia was maintained with inhaled isoflurane (1-2%) with periodic arterial blood gas monitoring. An intravenous line was placed using aseptic technique in the auricular marginal vein, and saline IV was administered, as necessary. The animal was intubated with an appropriate sized endotracheal tube, tied into place with cuff inflated to prevent leakage. ECG pads and electrodes are placed over the shaven metacarpal and metatarsal areas to monitor and record leads I, II, and III on the records. Vital signs (heart rate, respiratory rate, O₂ pulse oximeter, blood pressure) were monitored at approximately five minute intervals throughout the procedure. Heparin was administered to maintain an ACT of >300 seconds.

### Sheath Insertion

After the proper depth of anesthesia was obtained, the neck was prepared with providone-iodine followed by 70% ethanol. An incision was created using general sterile technique, and 8 Fr sheaths inserted into both the carotid artery and vein.

### Cardiac Function Testing

Baseline echocardiogram was performed.

### Creation of Mitral Valve Regurgitation

Through the carotid artery sheath, a forceps was advanced into the left ventricle (LV). Under fluoroscopic guidance, the forceps was used to cut cordae of the posterior papillary muscle to create severe mitral valve regurgitation (MR). Heart failure was defined as dilatation of left ventricular chamber and the drop of BP and raise of EDP following the confirmation of angiographically severe MR with left ventriculogram at Day 0.

### Recovery

Once vital signs were stable for 10 minutes after the completion of the procedure, the incision was closed, the isoflurane turned off, and medication administered for prevention of acute heart failure. Any potential pain was routinely relieved with buprenorphine 0.005-0.01 mg/kg given IM. The animals were also given cefazolin to prevent infection (1 g IV).

### Day 1-3 - Monitoring and Medication for Acute Heart Failure

Animals were checked three times per day for the first three days following MR creation. Animals were examined daily for signs of acute heart failure, wound infection at the neck incision site or any signs of pain or discomfort. Medication for acute heart failure was administered and recorded.

### Day 56 - Documentation of Heart Failure, Randomization, Blood Samples and Vehicle Control or AAV2/1/SERCA2a Administration

### Cardiac Function Testing

The images included: long axis, short axis, short axis M-mode at level of insertion of the papillary muscles and color Doppler of the mitral annulus. Cardiac output, Tau, ejection fraction, LV fractional shortening, LV free wall and septal dimensions, and MR assessment were computed and recorded.

### Group Assignment

Animals that survived to Day 56 (which met the definition of heart failure) were assigned to one of two groups: group 1, AAV2/1/SERCA2a at a dose of 1 x 10¹² DRP (n=4), or group 2, vehicle control (n=5).

### General Procedures: AAV2/1/SERCA2a or Vehicle Administration by Antegrade Epicardial Coronary Infusion (Groups 1-2)

On Day 56, all animals were administered a full dose of heparin to achieve an ACT>300. A coronary artery infusion catheter was used to infuse AAV2/1/SERCA2a or vehicle into the left coronary artery using a HCT infusion pump. The materials and procedures for implanting the catheter are described in detail in Example 2 and Tables 3 and 4 above. Essentially the same procedure was followed.

### Group 1: 1.0 x 10¹² AAV2/1/SERCA2a Administration (8 Minute Vector Infusion)

After gentle mixing, 0.56 mL of AAV2/1/SERCA2a stock solution was aseptically transferred to a sterile polypropylene tube and diluted with 10 mL of Formulation Buffer (130 mM NaCl, 20 mM HEPES and 1 mM MgCl₂ at pH 7.4), resulting in a concentration of 1 x 10¹¹ DRP/mL. Just prior to dosing the animal, 10 mL of diluted AAV2/1/SERCA2a (1 x 10¹¹ DRP/mL) was brought to room temperature and mixed with 10 mL of whole native blood from the animal being dosed, resulting in a final dilution in the syringe of 0.5 x 10¹¹ DRP/mL in a final volume of 20 mL. The infusion circuit was primed with blood and then the vector was delivered into the left main coronary artery over an 8 minute period, using the HCT infusion pump. Following completion of the infusion, the remaining solution was withdrawn from the tube connecting the end of the guiding catheter and the pump. This remaining solution was then infused manually over a period of approximately 2 minutes, followed by slow manual flush with 10 mL of saline.

### Group 2: Vehicle Administration (10 Minute Infusion)

The same procedure used for Group 1 described above was used for some of the animals in the vehicle (Formulation buffer ,130 mM NaCl, 20 mM HEPES and 1 mM MgCl₂ at pH 7.4) control group, with the exception that no AAV2/1/SERCA2a was administered. Other control group animals were administered Formulation buffer (130 mM NaCl, 20 mM HEPES and 1 mM MgCl₂ at pH 7.4) into the left coronary artery over 10 minutes using a V-Focus device (Preovolos AC, et al., J. Extra Corpor. Technol. 2006; 38(1):51-2). Both the antegrade epicardial coronary infusion method and the previously studied V-Focus Cardiac system deliver either test article or vehicle directly into the coronary arteries over a 10 minute period. There is no differences between no treatment control vs. control animals receiving vehicle administered via the V-Kardia Cardiac Delivery System.

### Day 112 - Terminal Sacrifice Procedures

### Anesthesia, Intubation and Anticoagulation

At Day 112 surviving animals were anesthetized, intubated and anticoagulated.

### Cardiac Function Testing

The images included: long axis, short axis, short axis M-mode at level of insertion of the papillary muscles and color Doppler of the mitral annulus. Cardiac output, Tau, ejection fraction, LV fractional shortening, LV freewall and septal dimensions, and MR assessment were computed and recorded. Tissue Doppler was also performed.

### Terminal Sacrifice

Animals were euthanized by use of cardioplegia solution and tissue samples were collected for protein and mRNA expression analysis.

### Preparation of swine heart microsomal fractions

The microsomal fraction, including SR vesicles, was prepared from frozen swine heart by the following method. About 5-10 g of heart muscle, cleaned of fat and connective tissue, were pulverized in liquid nitrogen, and homogenized in a buffer solution containing 5 mM Tris-HCl pH 7.4, 2 mM EDTA, and 8.5% sucrose with a Potter homogenizer. The homogenate was centrifuged at 1000×*g* for 10 min. The supernatant was then centrifuged for 15 min at 9000xg, and the resulting supernatant again twice for 15 min at 20 000×*g*. Sarcoplasmic reticulum vesicles present in this latter 20 000×*g* supernatant were subsequently pelleted by a 1 h 110 000×*g* spin. The pellet was resuspended in 500 µl of the homogenization buffer. All centrifugation steps were performed at 0-4°C.

### Preparation of protein extracts and immunoblotting analysis

Protein samples from normal untreated non-experimental control animals (control), AAV-SERCA2a transduced animals (SERCA2a), and Formulation buffer (saline) treated animals, were prepared from isolated swine microsomal fractions, matched for protein concentration (using the Bradford method) and were separated by SDS-PAGE and transferred onto nitrocellulose membranes. Membrane blots were incubated with antibodies against SERCA2a (Affinity Bioreagents, 1:400 dilution) overnight at 4°C. Reactive bands were visualized by chemiluminescence (PE Life Sciences) and films from at least three independent experiments were scanned and densities of the immunoreactive bands were evaluated using NIH Image software. Protein levels of GAPDH were used as an internal control. Density values of bands of SERAC2a were normalized against GAPDH values.

### RNA isolation and reverse transcriptase/polymerase chain reaction.

The mRNA levels of SERCA2a were measured using RT-PCR. Total RNA was isolated from normal untreated non-experimental control hearts (control), AAV-SERCA2a transduced heart (SERCA2a), and Formulation buffer (saline) treated heart, using TRIzol reagent (Invitrogen). After complete disruption of the tissue, chloroform was added, and the samples were shaken thoroughly before a brief incubation at room temperature. The samples were then centrifuged, and the supernatant (containing the RNA) was carefully removed without disturbing the cellular debris below it. RNA in the supernatant solution was precipitated by adding an equal volume of ice-cold isopropanol and pelleted by centrifugation at 12,000 g for 10 min at 4°C and washed by 75% ethanol. RNA pellets were resuspended in RNase-free water (Invitrogen). cDNA was synthesized from 1 µg of total RNA using iScript reverse transcriptase (Bio Rad) in a final volume of 20 µl. The level of GAPDH mRNA was evaluated as an internal control. The annealing temperature for PCR reaction cycles was adjusted according to the optimal annealing temperatures for each specific primer set.

### Results

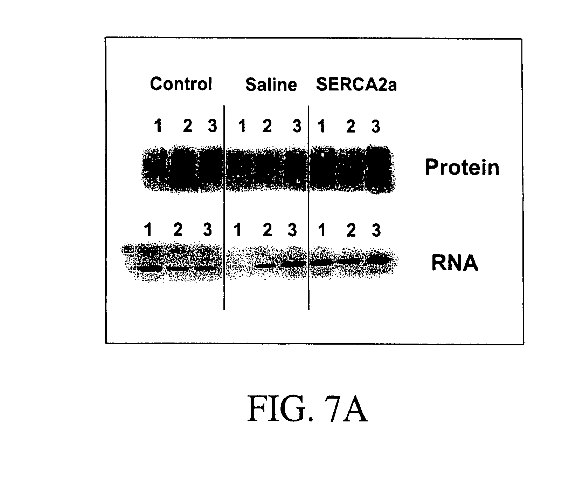

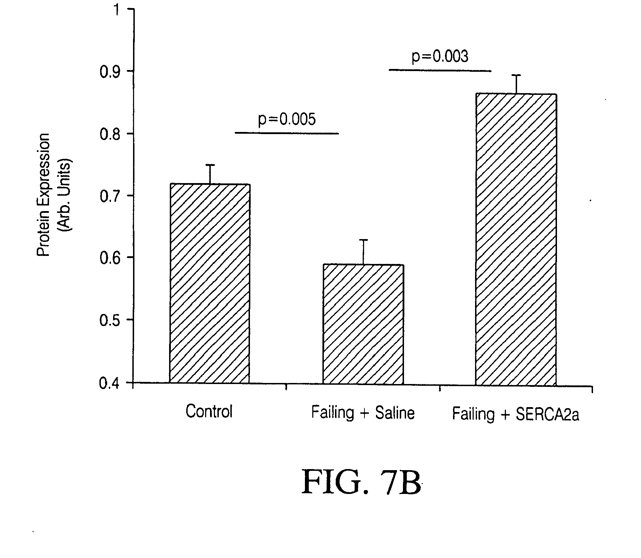

The results of the experiment are depicted in Figures 7-13. Figure 7A is polyacrylamide gels showing the expression of SERCA2a protein (top) and mRNA (bottom) in three non-experimental control animals, three formulation infused animals (saline) and three AAV2/1/SERCA2a treated animals. Figure 7B is a graph comparing the protein expression of SERCA2a between the three treatment groups, where the level of protein expression is normalized to GAPDH expression. This experiment shows that the AAV2/1/SERCA2a group had a higher level of SERCA2a mRNA and protein expression than either the non-experimental control group or the formulation buffer infused control group. In addition FIG 7B. shows that the normalized level of SERCA2a protein expression was statistically significantly higher in the SERCA2a infusion group that the experimental saline infusion control.

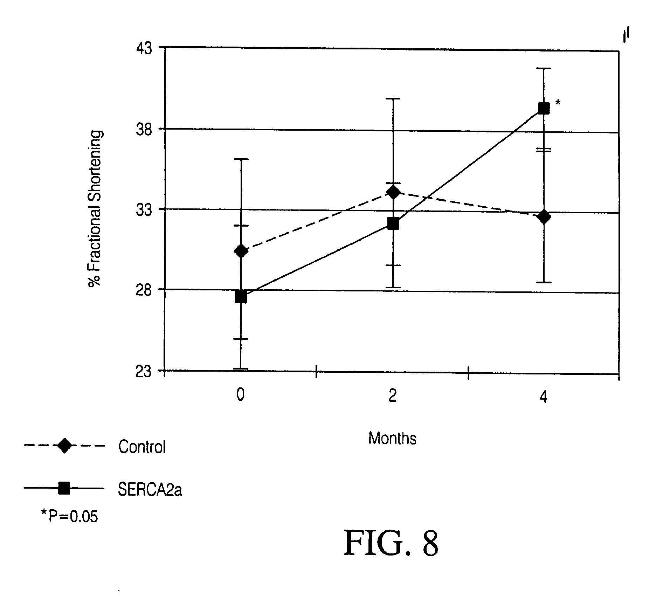

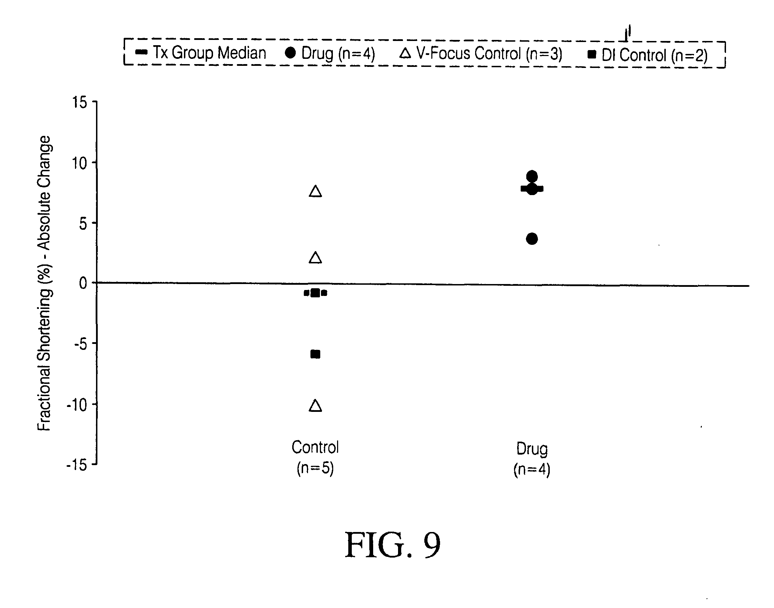

Figure 8 shows the percent fractional shortening of the left ventricle - a measure of the contractile function of the ventricle. Two months following administration of the AAV2/1/SERCA2a vector, the fractional shortening was increased by ~25% in the treatment group compared to the formulation buffer infused control group, a statistically significant improvement. Figure 9 is a plot of the absolute change in fractional shortening on day 112 compared to day 56. The median change of fractional shortening of the experimental formulation buffer control group (both V-Focus and direct infusion (DI) animals) is slightly negative, while the AAV2/1/SERCA2a infused group (drug) exhibits a substantial positive increase, indicating improved cardiac function.

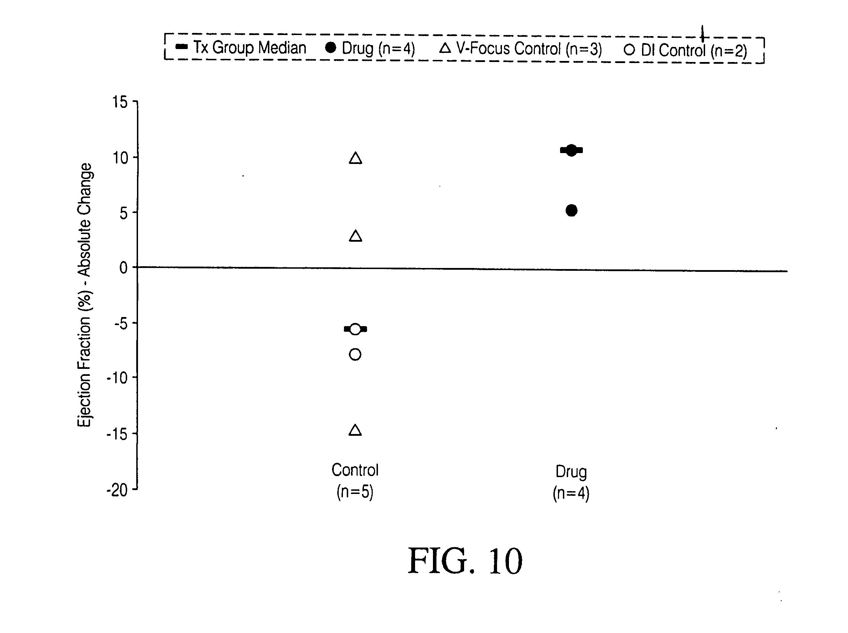

Similarly, Figure 10 is a plot of the absolute change in ejection fraction on day 112 compared to day 56. The median change in ejection fraction of the experimental formulation buffer control group (both V-Focus and direct infusion (DI) animals) is negative 5%, while the AAV2/1/SERCA2a infused group (drug) exhibits a substantial positive increase (about 10%), indicating improved cardiac function..

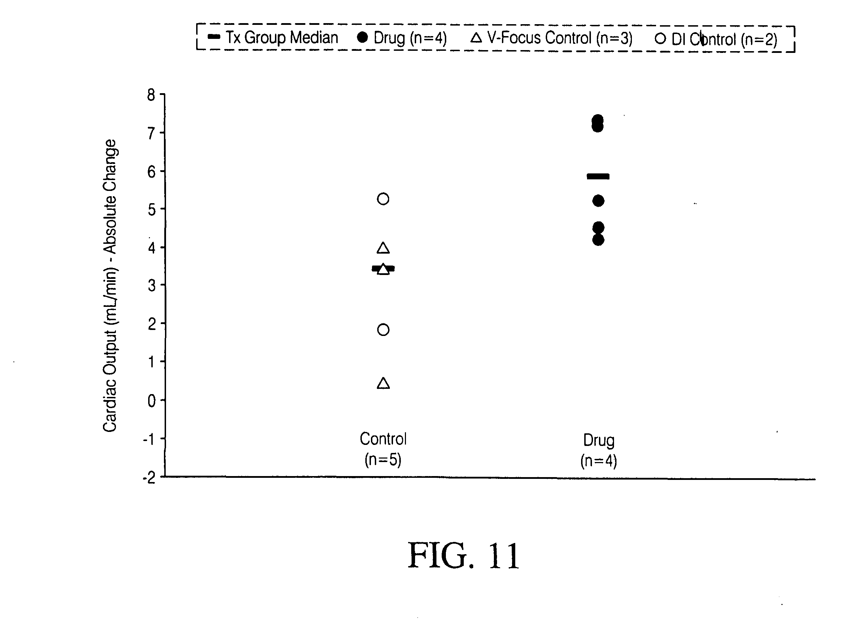

Figure 11 is a plot of the absolute change in cardiac output (mL/min.) on day 112 compared to day 56. The median change in cardiac output of the experimental formulation buffer control group (both V-Focus and direct infusion (DI) animals) is less than 3.5 mL/min., while the AAV2/1/SERCA2a infused group (drug) is nearly twice as much as the control group (about 6 mL/min.), indicating improved cardiac function..

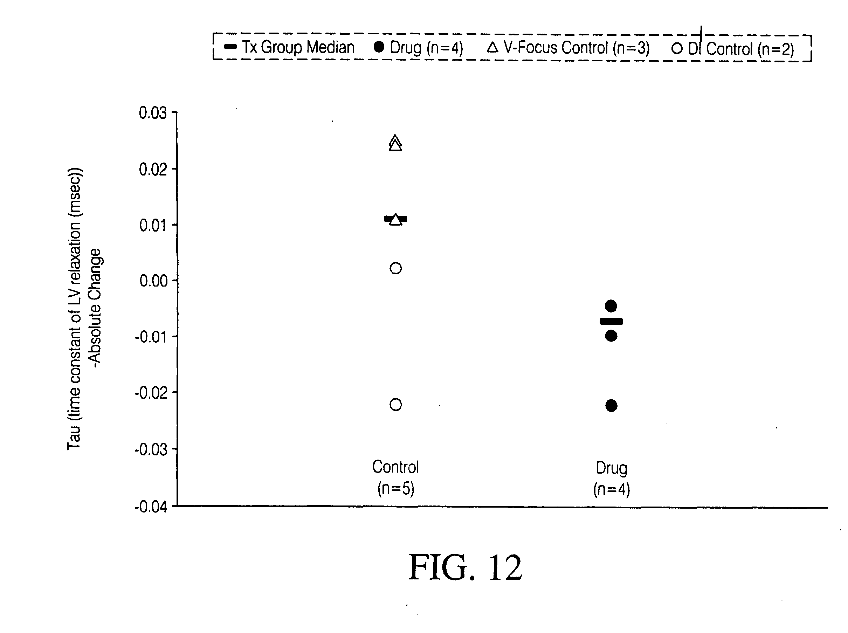

Figure 12 is a plot of the absolute change in tau (time constant of LV relaxation in msec.) on day 112 compared to day 56. Tau is a quantitative measure of diastolic performance requiring intraventricular pressure recording. The median change in relaxation period of the experimental formulation buffer control group (both V-Focus and direct infusion (DI) animals) is more than positive 0.01 msec., while the AAV2/1/SERCA2a infused group (drug) exhibits a substantial negative decrease (more than about 0.005 msec.), indicating improved cardiac function.

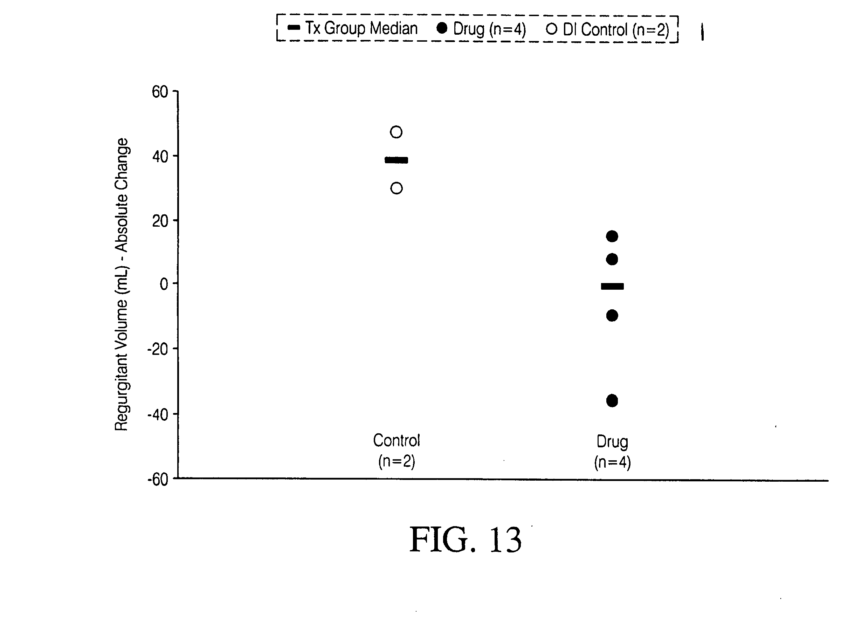

Finally, figure 13 is a plot showing the absolute change in regurgitant volume (mL) on day 112 compared to day 56. The median change in regurgitant volume of the experimental formulation buffer control group (direct infusion (DI) animals only) is nearly 40 mL, while the AAV2/1/SERCA2a infused group (drug) exhibits almost no change, indicating improved cardiac function compared to the control.

In addition, the left and right ventricles were both smaller in the AAV2/1/SERCA2a group as compared to the control groups (not shown), indicating less negative remodeling of the heart tissue due to heart failure in the treated group compared to the control group. Together, these results demonstrate that in an accepted animal model of heart failure, the antegrade epicardial extended infusion of a AAV2/1/SERCA2a vector successfully transfects heart tissue, resulting in increased expression of AAV2/1/SERCA2a mRNA and protein, as well as significant long-term improvements in several measures of heart function in an accepted large animal model of heart failure.

### Example 4: Anterograde Epicardial Infusion

The safety, feasibility and efficacy of a single intracoronary administration of 3 dose levels of AAV2/1/SERCA2a in patients with congestive heart failure is tested in a Phase 1, randomized, double-blinded, placebo-controlled dose escalation trial.

### Groups

The subject population is adult patients with NYHA Class III/IV chronic heart failure. Subjects are divided into four groups and receive either 3 x 10¹¹ DRP of AAV2/1/SERCA2a, 3 x 10¹² DRP of AAV2/1/SERCA2a, 1 x 10¹³ DRP of AAV2/1/SERCA2a, 3 x 10¹² DRP of AAV2/1/SERCA2a, or placebo. Subjects receiving AAV2/1/SERCA2a are followed for 12 months. Placebo subjects are unblinded after six months and offered AAV2/1/SERCA2a treatment.

### AAV2/1/SERCA2a Administration

The goal of the infusion is to provide diffuse, homogenous myocardial exposure to AAV2/1/SERCA2a via anterograde, epicardial coronary infusion. Multiple infusion scenarios exist based on collateralization patterns, occlusive disease, and anatomic variation (e.g. post surgical bypass anatomy), but the clinician's goal is 1/3 of AAV2/1/SERCA2a delivered to the anterolateral, 1/3 delivered to the posterolateral, and 1/3 delivered to the inferior/inferolateral myocardium.

Under local anesthesia (typically 1-2% lidocaine) and conscious sedation as appropriate per institutional standards, 6 Fr arterial (femoral, radial, or brachial per operator discretion) access is obtained using Seldinger technique. Unfractionated heparin is administered intravenously/intraarterially to provide activated clotting time (ACT) of 250-300 sec. Coronary and bypass graft angiography is performed in usual manner if not performed in prior 2 months. Anatomy is defined and strategy in place prior to AAV2/1/SERCA2A administration to accomplish homogenous delivery to the perfused myocardium and appropriate 6Fr guidecatheters are selected. Only one or two infusions are performed and the arteries or bypass grafts utilized for infusion are the one or two that subserve the largest portion of myocardial flow. Two-thirds of the AAV2/1/SERCA2A product is infused in the first infusion sequence, and 1/3 in the second (final) infusion sequence if needed. The selected, appropriate guidecatheter for the first infusion is engaged. in the first coronary artery/bypass graft (subtending the largest territory) after backbleeding/flushing in usual fashion.

A MEDRAD infusion system (City, PA) or equivalent and 60 cc syringe is filled with the AAV2/1/SERCA2A mixture (see below) in a sterile field. The guidecatheter is connected to 30" high flow pressure line male-female tubing assembly and to the Medrad power injector system to assure that a fluid-fluid air-free space is secured. Blood is aspirated and mixed with normal saline, followed by addition of AAV2/1/SERCA2A to provide the following mixtures in the MEDRAD power injector syringe. It is important that the blood/saline solution is added first to the syringe, followed by addition of AAV2/1/SERCA2A:
Low dose cohort: 3 x 10¹¹ DRP AAV2/1/SERCA2A
● 15 mL blood
● 45 ml normal saline for injection
● 0.3 ml of a 1 x 10¹² AAV2/1/SERCA2A DRP/mL solution
Mid-dose cohort: 3 x 10¹² DRP AAV2/1/SERCA2A
● 15 mL blood
● 42 ml normal saline for injection
● 3.0 ml of a 1 x 10¹² AAV2/1/SERCA2A DRP/mL solution
High-dose cohort: 1 x 10¹³ DRP AAV2/1/SERCA2a
● 15 mL blood
● 35 ml normal saline for injection
● 10.0 ml of a 1 x 10¹² AAV2/1/SERCA2A DRP/mL solution each)
Placebo cohort:
● 15 mL blood
● 45 ml normal saline for injection

### Single infusion procedure

The 60 mL of solution is infused at a constant flow rate of 6 mL/min. Final angiography is performed to evaluate interim anatomic changes with the infusion. The guidecatheter is withdrawn. Femoral sheath removal and/or closure is performed per operator discretion.

### Dual infusion procedure

In subjects receiving the infusion into two arteries/graphs, the first infusion is a 40 mL volume at a constant flow rate of 6 mL/min. The goal is to infuse 2/3 of the AAV2/1/SERCA2A product to the larger myocardial territory. After the first portion of the infusion, final angiography is performed to evaluate interim anatomic changes with the infusion. The guidecatheter is withdrawn.

Next, an appropriate 6Fr guidecatheter is chosen for the infusion sequence to the minor (1/3) perfused myocardial territory. The guidecatheter is engaged into the artery/bypass graft and connected to the Medrad injection assembly as per prior description, and the final 1/3 of the AAV2/1/SERCA2A product is infused (20 mL volume, at a constant rate of 6 mL/min.). Final angiography is performed to evaluate interim anatomic changes with the infusion. The guidecatheter is withdrawn. Femoral sheath removal and/or closure is performed per operator discretion.

### Anatomic vignettes

Multiple anatomic scenarios can exist that determine arterial choice for infusion to achieve 2/3 delivery of product to the "major" perfused myocardial territory and 1/3 infused to the "minor" territory:
(1) *Nonobstructive coronary disease*/*normal coronaries, no prior coronary bypass surgery (CABG)* - standard left main coronary guidecatheter engagement with 2/3 infusion into the left coronary (left anterior descending and left circumflex) systems; standard right coronary (RCA) guidecatheter engagement and 1/3 infusion into the RCA system.
(2) *No prior CABG, RCA total occlusion, left to right coronary collateralization* - left main guidecatheter engagement, 100% of the product is delivered to infuse all (LAD, LCx, and RCA) territories.
(3) *No prior CABG, LAD and*/*or LCx total occlusion, right-to-left collaterals from RCA* - RCA becomes "major" territory in this circumstance and 2/3 product is delivered to RCA, 1/3 to left coronary system.
(4) *Multivessel occlusive disease, no prior CABG* - 2/3 product is delivered to territory serving the majority of myocardium, 1/3 to minor
(5) *Prior CABG, patent grafts* - 2/3 is delivered into graft to LAD, 1/3 is delivered into graft to RCA
(6) *Prior CABG, mixed occlusive bypass graft and native coronary disease* - native or bypass graft 2/3 infusion into the vessel that supplies the largest myocardial territory, 1/3 into the minor territory.

It should be emphasized that anatomic variants will differ between individuals, but for a typical individual, the preferred embodiment is to deliver 2/3 of product to the major perfused territory and 1/3 to the minor (exception is single 100% product infusion if a single native artery or bypass graft supplies the majority of myocardial flow).

### Assessment of Cardiac Function

The primary activity/efficacy endpoints that are evaluated and compared within and between treatment groups based on changes from baseline compared to 3, 6 and 12 months following AAV2/1/SERCA2a administration include one or more of the following: VO₂ max assessed by cardiopulmonary exercise testing; echocardiographic assessments including left ventricular ejection fraction, LV dimensions, regional wall motion, diastolic function, and mitral regurgitation; distance walked during the 6-minute walk test; NYHA classification; and B-Type Natriuretic Peptide (BNP) level.

### Results

A substantial and significant improvement in one or more of the above activity/efficacy endpoints is seen in the treatment groups compared to the placebo group. The following paragraphs describe embodiments of the invention.
1. Use of a therapeutic polynucleotide composition adapted to transfect cardiac cells in the preparation of a medicament for treating or preventing cardiac disease by infusing the polynucleotide into a blood vessel of the coronary circulation over a period of at least three minutes, without isolating the coronary circulation from the systemic circulation.
2. The use of paragraph 1, wherein said polynucleotide is infused into said blood vessel over a period of at least five minutes.
3. The use of paragraph 1, wherein said polynucleotide is infused into said blood vessel over a period of at least ten minutes.
4. The use of paragraph 1, wherein said polynucleotide is infused into said blood vessel over a period of at least fifteen minutes.
5. The use of paragraph 1, wherein said infusion into said blood vessel is at a rate of less than or equal to 6.0 mL/min.
6. The use of paragraph 1, wherein said infusion into said blood vessel is at a rate of less than or equal to 2.5 mL/min.
7. The use of paragraph 6, wherein said infusion into said blood vessel is at a rate of less than or equal to 2.0 mL/min.
8. The use of paragraph 6, wherein said infusion into said blood vessel is at a rate of less than or equal to 1.2 mL/min.
9. The use of paragraph 6, wherein said infusion into said blood vessel is at a rate of less than or equal to 1.0 mL/min.
10. The use of paragraph 6, wherein said infusion into said blood vessel is at a rate of less than or equal to 0.6 mL/min.
11. The use of paragraph 1, wherein said blood vessel is the left coronary artery.
12. The use of paragraph 1, wherein the outflow of the coronary circulation is not nonnaturally restricted.
13. The use of paragraph 11, wherein transfection of cardiac cells of the anterior lateral ventricle, inferior lateral ventricle, septum and right ventricle is detectable using PCR.
14. The use of paragraph 1, wherein the polynucleotide is capable of expressing a protein capable of modulating a cellular activity of the cardiac cells.
15. The use of paragraph 14, wherein said cellular activity is a calcium cycling pathway of a cardiomyocyte.
16. The use of paragraph 15, wherein said protein is a sarcoplasmic/endoplasmic reticulum ATPase (SERCA).
17. The use of paragraph 16, wherein the SERCA is SERCA2a.
18. The use of paragraph 1, wherein said polynucleotide is present in a viral vector selected from the group consisting of an adeno-associated virus, an adenovirus, a retrovirus, a herpes simplex virus, a bovine papilloma virus, a lentiviral vector, a vaccinia virus, and a polyoma virus.
19. The use of paragraph 18, wherein said viral vector is AAV virus.
20. The use of paragraph 18, wherein said viral vector is an AAV2/1 vector.
21. The use of paragraph 20, wherein said polynucleotide is operably linked to a CMV-based promoter and packaged in said viral vector.
22. The use of paragraph 21, wherein said polynucleotide comprises a SERCA2a coding sequence.
23. The use of paragraph 22, wherein said transfection of said cardiac cells increases lateral ventricle fractional shortening.
24. The use of paragraph 22, said disease is congestive heart failure.
25. The use of paragraph 1, wherein said polynucleotide is packaged in a DNase resistant particle (DRP) of a viral vector.
26. The use of paragraph 25, wherein the total number of DRP infused is less than or equal to an amount selected from the group consisting of 1 x 10¹⁴, 1 x 10¹³, 3 x 10¹², 1 x 10¹², 1 x 10¹¹, 1 x 10¹⁰, 1 x 10⁹, and 1 x 10⁸.
27. The use of paragraph 26, wherein the total number of DRP infused is less than or equal to 1 x 10¹³.
28. The use of paragraph 27, wherein the total number of DRP infused is less than or equal to 1 x 10¹², the viral vector is AAV2/1, the polynucleotide comprises a SERCA2a coding sequence, said blood vessel is the left or right coronary artery, and said infusion of said polynucleotide lasts at least 10 minutes at a flow rate of less than or equal to 2 mL/min; and wherein transfection of cardiac cells of the anterior lateral ventricle, inferior lateral ventricle, septum and right ventricle is detectable using PCR.
29. The use of paragraph 1, wherein said disease is selected from the group consisting of heart failure, ischemia, arrhythmia, myocardial infarction, congestive heart failure, transplant rejection, abnormal heart contractility, non-ischemic cardiomyopathy, mitral valve regurgitation, aortic stenosis or regurgitation, abnormal Ca²⁺ metabolism and congenital heart disease.
30. The use of paragraph 27, wherein the viral vector is AAV2/1, the polynucleotide comprises a SERCA2a coding sequence, said blood vessel is the left or right coronary artery, and said infusion of said polynucleotide lasts at least 8 minutes at a flow rate of less than or equal to 2.5 mL/min; and
   wherein transfection of cardiac cells of the anterior lateral ventricle, inferior lateral ventricle, septum and right ventricle is detectable using PCR; and
   wherein said transfection of said cardiac cells increases lateral ventricle fractional shortening when measured 4 months after said infusion by at least 25% as compared to lateral ventricle fractional shortening before infusion of the polynucleotide.
31. The use of paragraph 27, wherein the viral vector is AAV2/1, the polynucleotide comprises a SERCA2a coding sequence, said blood vessel is the left or right coronary artery, and said infusion of said polynucleotide lasts at least 10 minutes.
32. The use of paragraph 31, wherein the disease of the cardiovascular system is congestive heart failure.
33. The use of paragraph 31, wherein said infusion of said polynucleotide is at a flow rate of 6 mL/min.
34. The use of paragraph 31, wherein said transfection of said cardiac cells results in an improvement in a measure of cardiac function selected from the group consisting of expression of SERCA2a protein, fractional shortening, ejection fraction, cardiac output, time constant of ventricular relaxation, and regurgitant volume.
35. The use of paragraph 27, wherein said medicament is infused into an additional blood vessel of the coronary circulation over a period of at least three minutes.
36. The use of paragraph 35, wherein the viral vector is AAV2/1, the polynucleotide comprises a SERCA2a coding sequence;
   wherein said blood vessel is the left coronary artery, and said infusion of said polynucleotide into the left coronary artery lasts at least 6 minutes; and
   wherein said additional blood vessel is the right coronary artery, and said infusion of said polynucleotide into said right coronary artery lasts at least 3 minutes.
37. The use of paragraph 36, wherein the disease of the cardiovascular system is congestive heart failure.
38. The use of paragraph 36, wherein said infusion of said polynucleotide into said left coronary artery and said right coronary artery is at a flow rate of 6 mL/min.
39. The use of paragraph 36, wherein said transfection of said cardiac cells results in an improvement in a measure of cardiac function selected from the group consisting of expression of SERCA2a protein, fractional shortening, ejection fraction, cardiac output, time constant of ventricular relaxation, and regurgitant volume.
40. A method of treating or preventing a cardiovascular disease by transfecting cardiac cells of a large mammal, the method comprising:
   identifying an mammal in need of treatment or prevention of a cardiovascular disease;
   infusing a therapeutic polynucleotide into a blood vessel of the coronary circulation in vivo;
   wherein said therapeutic polynucleotide is infused into said blood vessel over a period of at least three minutes;
   wherein the coronary circulation is not isolated or substantially isolated from the systemic circulation of the mammal; and
   wherein said therapeutic polynucleotide transfects cardiac cells of said mammal resulting in the treatment or prevention of said cardiovascular disease.
41. The method of paragraph 40, wherein said polynucleotide is packaged in a DNase resistant particle (DRP) of a viral vector, the total number of DRP infused is less than or equal to 1 x 10¹³ and the viral vector is AAV2/1, the polynucleotide comprises a SERCA2a coding sequence, said blood vessel is the left or right coronary artery, and said infusion of said polynucleotide lasts at least 10 minutes.
42. The method of paragraph 41, wherein the disease of the cardiovascular system is congestive heart failure.
43. The method of paragraph 41, wherein said transfection of said cardiac cells results in an improvement in a measure of cardiac function selected from the group consisting of expression of SERCA2a protein, fractional shortening, ejection fraction, cardiac output, time constant of ventricular relaxation, and regurgitant volume.
44. A composition comprising a therapeutic polynucleotide adapted to transfect cardiac cells for treating or preventing cardiac disease by infusing the polynucleotide into a blood vessel of the coronary circulation over a period of at least three minutes, without isolating the coronary circulation from the systemic circulation.
45. The composition of paragraph 44, wherein said polynucleotide comprises a SERC A2a coding sequence packaged in a DNase resistant particle (DRP) of an AAV2/1 viral vector, the total number of DRP infused is less than or equal to 1 x 10¹³, said blood vessel is the left or right coronary artery, and said infusion of said polynucleotide lasts at least 10 minutes.
46. The composition of paragraph 45, wherein the disease of the cardiovascular system is congestive heart failure.
47. A kit comprising: a pharmaceutical composition comprising at least 1 x 10¹¹ DRP of AAV2/1 vector containing a polynucleotide encoding for SERCA2a; and
   instructions instructing that a solution comprising at least 0.5 x 10¹¹ DRP of AAV2/1 vector containing a polynucleotide encoding for SERCA2a should be administered to a patient in need of prevention or treatment of a cardiovascular disease by in vivo infusion into a blood vessel of the coronary circulation wherein said coronary circulation is not isolated or substantially isolated from the systemic circulation of the patient, and wherein said polynucleotide is infused into said blood vessel over a period of at least three minutes.
48. The kit of paragraph 47, wherein said blood vessel is the left or right coronary artery, and said infusion of said polynucleotide is for a period of at least eight minutes.

## Claims

1. A therapeutic polynucleotide composition adapted to transfect cardiac cells for use in treating or preventing cardiac disease, wherein said treatment or prevention of cardiac disease is by infusing the polynucleotide into a blood vessel of the coronary circulation over a period of at least three minutes, without isolating or without substantially isolating the coronary circulation from the systemic circulation.

2. A therapeutic polynucleotide composition according to claim 1 for the use of claim 1, wherein the outflow of the coronary circulation is not nonnaturally restricted.

3. A therapeutic polynucleotide composition according to claim 1 for the use of claim 1 or 2, wherein said disease is congestive heart failure.

4. A therapeutic polynucleotide composition according to claim 1 for the use of any one of claims 1 to 3, wherein said blood vessel is the left coronary artery or the right coronary artery.

5. A therapeutic polynucleotide composition according to claim 1 for the use of any one of claims 1 to 4, wherein said infusion into said blood vessel is at a flow rate of less than or equal to 10.0 mL/min.

6. A therapeutic polynucleotide composition according to claim 1 for the use of any one of claims 1 to 5, wherein transfection of cardiac cells of the anterior lateral ventricle, inferior lateral ventricle, septum and right ventricle by the therapeutic polynucleotide is detectable using PCR.

7. A therapeutic polynucleotide composition according to claim 1 for the use of any one of claims 1 to 6, wherein said polynucleotide is packaged in a DNase resistant particle (DRP) of a viral vector, and the total number of DRP infused is less than or equal to 1 x 10¹⁴.

8. A therapeutic polynucleotide composition according to claim 1 for the use of claim 7, wherein said viral vector is an adeno-associated virus (AAV).

9. A therapeutic polynucleotide composition according to claim 1 for the use of claim 8, wherein said viral vector is an AAV1 vector.

10. A therapeutic polynucleotide composition according to claim 1 for the use of claim 8, wherein said AAV viral vector comprising heterologous capsid proteins such that capsid proteins VP1, VP2 and VP3 are not all of the same serotype AAV.

11. A therapeutic polynucleotide composition according to claim 1 for the use of claim 8, wherein said viral vector is an AAV2/1 vector.

12. A therapeutic polynucleotide composition according to claim 1 for the use of any one of claims 7 to 11, wherein said polynucleotide is operably linked to a CMV-based promoter and packaged in said viral vector.

13. A therapeutic polynucleotide composition according to claim 1 for the use of any one of claims 1 to 12, wherein said polynucleotide comprises a SERCA2a coding sequence.

14. A therapeutic polynucleotide composition according to claim 1 for the use of claim 7, wherein said viral vector is AAV2/1, said polynucleotide comprises a SERCA2a coding sequence, said blood vessel is the left, the right, or the left and the right coronary artery, said infusion of said polynucleotide lasts at least 8 minutes, and said flow rate of said infusion is less than or equal to 6.0 mL/min.

15. A therapeutic polynucleotide composition according to claim 1 for the use of any one of claims 1 to 14, wherein said transfection of said cardiac cells increases lateral ventricle fractional shortening when measured 4 months after said infusion as compared to lateral ventricle fractional shortening before infusion of the polynucleotide.
